# EUROPEAN PATENT APPLICATION

(11) **EP 4 089 090 A1**
(43) Date of publication of application: **16.11.2022**
(21) Application number: 20910574.1
(22) Date of filing: 30.12.2020
(51) Int. Cl.: C07D 413/06

(54) **METHOD FOR PREPARING COUMARIN COMPOUND, 3 POSITION OF WHICH IS SUBSTITUTED WITH AMIDOALKYL AND PRODUCTS AND RELATED INTERMEDIATES THEREOF**

(30) Priority: 31.12.2019 CN 201911422365
(71) Applicant: Fosun Orinove Pharmatech, Inc., Suzhou, Jiangsu 215123 (CN)
(72) Inventor: ZENG, Qingping, Thousand Oaks, California 91320 (US); WANG, Ruiping, Beijing 100176 (CN); DUAN, Jie, Beijing 100176 (CN); WEI, Xudong, Coventry, Rhode Island 02816 (US)
(74) Representative: Müller, Christian Stefan Gerd
(86) International application number: PCT/CN2020/141203
(87) International publication number: WO 2021/136333

(57) **Abstract**

The present invention relates to a method for preparing coumarin compounds substituted by amidoalkyl at 3-position, and related intermediates thereof. The method includes:
Step (A): reacting a compound of formula (VI) with a compound of formula (V) to give a compound of formula (VII) 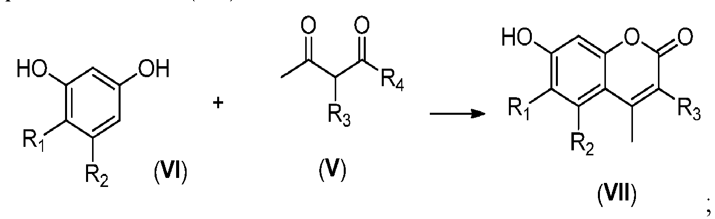 wherein R₁ and R₂ are each independently selected from hydrogen, halogen, and -O(C₁₋₈ alkyl), wherein the alkyl is optionally substituted with 1 or 2 groups selected from the group consisting of halogen, C₁₋₈ alkyl substituted with 1-2 hydroxyl groups, -O(C₁₋₈ alkyl);
R₃ is -L-C(O)NR₅R₆, L is C₁₋₃ alkylene.

## Description

This application claims the priority of Chinese application No. 201911422365.2 filed on December 31, 2019, entitled "a method for preparing coumarin compounds substituted by amidoalkyl at 3-position, the products and related intermediates thereof". The entire text of the Chinese application is incorporated herein by reference.

### Technical Field

The present invention relates to the field of medicinal chemistry, and particularly relates to a method for preparing coumarin compounds substituted by amidoalkyl at 3-position, the products and related intermediates thereof.

### Background

Cancers or tumors are the second leading cause of death in the developed world, and despite continuous emergence of advanced early detection techniques, new treatments and improved treatment outcomes, new drugs and treatments are still needed to improve patients' life quality. Inositol-requiring enzyme 1α (IRE-1α) is an endonuclease, mainly located in the endoplasmic reticulum connected to the nucleus. It regulates gene expression by exerting the catalytic function of endonuclease, thereby affecting the processing and modification of unfolded proteins in the endoplasmic reticulum. When cells are stimulated by negative factors such as hypoxia, starvation, inflammation, viral infection, carcinogenesis, and are in a state of imbalance in the internal environment, endoplasmic reticulum stress will occur, causing protein synthesis and folding disorders and eventually leading to cell apoptosis. At this point a cellular mechanism of self-protection called the "unfolded protein response" (UPR) will be activated and act against endoplasmic reticulum stress: as an important part of the signaling pathway of this mechanism, IRE-1α will be activated. It regulates the expression of related genes by excising a specific base sequence on the target mRNA, thereby enhancing protein folding and modification, relieving endoplasmic reticulum stress, repairing the imbalanced state of the intracellular environment and promoting cell survival. Therefore, the function of IRE-1α is closely related to diseases in many fields such as tumor, metabolism, immunity, viral infection, and cardiovascular. In particular, for cancer, the massive and rapid proliferation of malignant tumor cells causes a general state of out-of-control conditions for the tumor cells, with interaction of various factors, such as hypoxia, insufficient nutrient supply, metabolic disorders, and carcinogenic pressure. Such tumor microenvironment continues to affect endoplasmic reticulum folding; in addition, chemotherapy, biological therapy and radiation therapy can also induce endoplasmic reticulum stress response and resist apoptosis through UPR, so IRE-1α in tumor cells is continuously and extensively activated under long-term stress, making it an ideal tumor target that can be acted upon by drugs such as IRE-1α inhibitors.

CN103079558 A discloses a class of small-molecule compounds with a coumarin core structure and can inhibit IRE-1α, wherein the compounds with the following specific structures are provided. It is a potent, low toxicity and highly selective IRE-1α inhibitor.

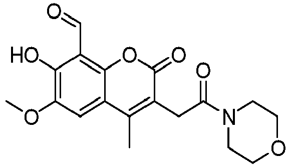

CN107973784 A discloses the following synthetic route of a ethyl acetoacetate compound (ethyl 2-(*N*,*N*-dimethylaminocarbonylmethyl)acetoacetate), which is substituted by amidoalkyl group at α-position:

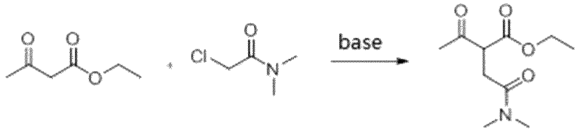

The 1,3-dicarbonyls in the ethyl acetoacetate structure of the compound undergo a condensation reaction with an iminoamide derivative under basic conditions, and is used for synthesizing a pyrimidine compound

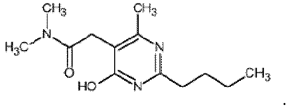

### Summary

The first aspect of the present invention provides a method for the preparation of a compound of formula (VII), comprising

Step (A): reacting a compound of formula (VI) with a compound of formula (V) to give a compound of formula (VII) 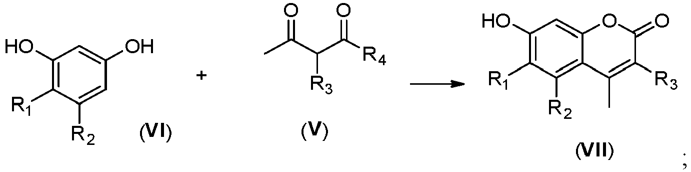 wherein R₁ and R₂ are each independently selected from the group consisting of hydrogen, halogen, and -O(C₁₋₈ alkyl), wherein the alkyl is optionally substituted with 1 or 2 groups selected from the group consisting of halogen, C₁₋₈ alkyl substituted with 1-2 hydroxyl groups, -O(C₁₋₈ alkyl);
R₃ is -L-C(O)NR₅R₆, L is C₁₋₃ alkylene;
R₄ is -O(C₁₋₆ alkyl);
R₅ and R₆ are each independently selected from the group consisting of hydrogen, -O(C₁₋₈ alkyl), C₃₋₁₀ cycloalkyl, wherein the alkyl, cycloalkyl are optionally substituted with 1 or 2 groups selected from the group consisting of halogen, -O(C₁₋₈ alkyl), -NH₂, -NH(C₁₋₈ alkyl), - N(C₁₋₈ alkyl)₂, five- to seven-membered heterocyclyl, wherein the heterocyclyl contains 1-2 heteroatoms selected from the group consisting of N, O, S, and the heterocyclyl is optionally substituted with 1-2 groups selected from the group consisting of C₁₋₈ alkyl; or
R₅ and R₆ together with the N atom to which they are attached form a five- to seven-membered heterocyclyl, and the heterocyclyl optionally contains 1 additional heteroatom selected from the group consisting of N, O, S, and is optionally substituted with 1 or 2 groups selected from the group consisting of halogen, C₁₋₈ alkyl, -O(C₁₋₈ alkyl), -NH₂, -NH(C₁₋₈ alkyl), -N(C₁₋₈ alkyl)₂.

The second aspect of the present invention provides a method for the preparation of a compound of formula (I), comprising step (A) described in the first aspect of the present invention and the following step (B):

### Step (B): preparing the compound of formula (I) from the compound of formula (VII)

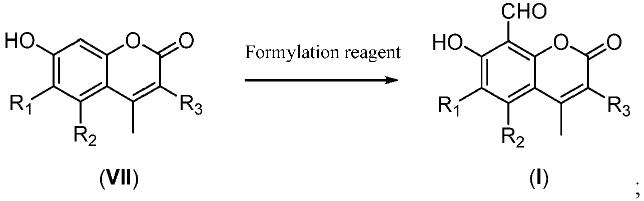

wherein R₁, R₂ and R₃ are as defined in the first aspect of the present invention.

The third aspect of the present invention provides an intermediate compound, and the structure is shown in the following formula (V): 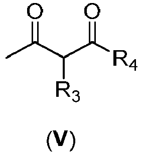 wherein R₃ and R₄ are as defined in the first aspect of the invention.

The fourth aspect of the present invention provides a crystal form I of the compound Orin1001, wherein the X-ray powder diffraction pattern of the crystal form I has characteristic peaks at diffraction angles (2θ) of about 8.44±0.2°, 13.11±0.2°, 15.70±0.2°, 19.73±0.2°, 21.00±0.2° and 22.91±0.2°,

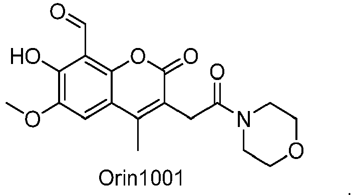

In yet another aspect, the present invention provides a pharmaceutical composition comprising the crystal form I of the present invention and one or more pharmaceutically acceptable carriers.

In yet another aspect, the present invention also provides the crystal form I of the present invention or the pharmaceutical composition of the present invention in the manufacture of a medicament for the treatment or prevention of a disease, disorder or condition associated with the unfolded protein response (UPR), or the use in the manufacture of a medicament for the treatment or prevention of a disease, disorder or condition associated with a target of regulated IRE1-dependent decay (RIDD).

### Brief Description of the Drawings

Figure 1 shows the X-ray powder diffraction (XRPD) pattern of the crystal form I.
Figure 2 shows the thermogravimetric analysis (TGA) pattern of the crystal form I.
Figure 3 shows the Differential Scanning Calorimetry (DSC) pattern of the crystal form I.
Figure 4 shows the Dynamic Vapor Sorption (DVS) pattern of the crystal form I.
Figure 5 shows the XRPD comparison chart of crystal form I before and after DVS detection.
Figure 6 shows results of crystal form I stability test (XRPD) - Day 7, wherein "-1" and "-2" at the end of the annotation of the curve indicate "Sample-1" and "Sample-2", respectively.
Figure 7 shows results of crystal form I stability test (XRPD) - Day 14, wherein "-1" and "-2" at the end of the annotation of the curve indicate "Sample-1" and "Sample-2", respectively.
Figure 8 shows a graph of particle size distribution test of the crystal form I.
Figure 9 shows the test results (XRPD) of the crystal form I after mechanical treatment.

### Detailed Description

### General terms and definitions

Unless stated otherwise, all technical and scientific terms used herein have the same meanings identical to those understood by one of ordinary skill in the art to which this invention pertains. In the case of contradiction, the definitions provided herein will prevail. The trade name used herein refers to the corresponding commercial product or the active ingredient. All patents, published patent applications, and reference cited herein are incorporated herein by reference.

When used with a numerical variable, the term "approximate" or "about" usually refers to the value of the variable and all the values of the variable within the experimental error (for example, within an average 95% confidence interval) or within ± 10% of the specified value, or a wider range.

The term "optional" or "optionally" means the event described subsequent thereto may or may not happen. This term encompasses the cases that the event may or may not happen.

The terms "substitution" and "substituted" mean that one or more (e.g., one, two, three, or four) hydrogens on the designated atoms are replaced by a selection from the indicated groups, provided that the designated atom's normal valency under the existing circumstances is not exceeded, and that the substitution results in a stable compound. Combinations of substituents and/or variables are permissible only if such combinations form stable compounds. When a substituent is described as being absent, it should be understood that there may be one or more hydrogen atoms at the position of the substituent, provided that the resulting structure enables the compound to reach a stable state.

If a substituent is described as "optionally substituted," the substituent may be unsubstituted, or it may be substituted. If an atom or group is described as being optionally substituted with one or more substituents from a list of substituents, one or more hydrogens on the atom or group may each be replaced by optional substituent(s), wherein the optional substituent(s) is/are independently selected . When the substituent is oxo (i.e. =O), it means that two hydrogen atoms are substituted.

Unless otherwise indicated, as used herein, a substituent can be attached at any suitable position thereof.

The expression "comprise" or its synonyms "include", "contain" and "have" and the like are open-ended and do not exclude additional unlisted elements, steps or ingredients. The expression "consist of" excludes any unlisted element, step or ingredient. The expression "substantially consist of' refers to specified elements, steps or ingredients within a given range, together with optional elements, steps or components which do not substantively affect the basic and novel feature of the claimed subject matter. It should be understood that the expression "comprise" encompasses the expressions "substantially consist of" and "consist of".

The term "one or more" or "at least one" may mean one, two, three, four, five, six, seven, eight, nine or more.

When the lower and upper limits of a numerical range are disclosed, any numerical value and any inclusive range falling within that range is specifically disclosed. In particular, every range of values disclosed herein should be understood to mean every number and range that falls within the broader range.

The expression m-n as used herein refers to the range of m to n and the sub-ranges formed by each point-value therein and each point-value therein. For example, the expression "C₁₋₈" covers a range of 1-8 carbon atoms and should be understood to also cover any sub-range therein and every point-value, e.g., C₂₋₅, C₃₋₄, C₁₋₂, C₁₋₃, C₁₋₄, C₁₋₅, C₁₋₆, C₁₋₇, etc., and C₁, C₂, C₃, C₄, C₅, C₆, C₇, C₈, etc. For example, the expression "C₃₋₁₀" should also be understood in a similar manner, e.g., can encompass any sub-range and point-value contained therein, e.g., C₃₋₉, C₆₋₉, C₆₋₈, C₆₋₇, C₇₋₁₀, C₇₋₉, C₇₋₈, C₈₋₉, etc. and C₃, C₄, C₅, C₆, C₇, C₈, C₉, C₁₀, etc. For another example, the expression "three- to ten-membered" should be understood to cover any sub-range and each point-value therein, such as 3-5 membered, 3-6 membered, 3-7 membered, 3-8 membered, 4-5 membered, 4-6 membered, 4-7 membered, 4-8 membered, 5-7 membered, 5-8 membered, 6-7 membered, 7-8 membered, 9-10 membered, etc., as well as 3, 4, 5,6, 7, 8, 9, 10-membered, etc. Other similar expressions herein should also be understood in a similar manner.

Unless otherwise indicated herein, reference to singular forms such as "a", "an", "the" include reference to plural forms.

The term "halo" or "halogen" or "halogenated" is to be understood to mean a fluorine (F), chlorine (Cl), bromine (Br) or iodine (I) atom, preferably a fluorine, chlorine or bromine atom.

The term "alkyl", as used herein alone or in combination with other groups, refers to a saturated straight chain, branched chain or cyclic hydrocarbon group. As used herein, the term "C₁₋₈ alkyl" refers to a saturated straight, branched or cyclic hydrocarbon group having 1-8 (e.g., 1, 2, 3, 4, 5, 6, 7, or 8) carbon atoms. For example, "C₁₋₆ alkyl" can be methyl, ethyl, n-propyl, isopropyl, n-butyl, isobutyl, sec-butyl, tert-butyl, n-pentyl, isopentyl, neopentyl, 3-methylpentan-3-yl, hexyl (e.g., n-hexyl, cyclohexyl, etc.). "C₁₋₈ alkyl" encompasses sub-ranges therein such as "C₁₋₃ alkyl", "C₂₋₃ alkyl", "C₄₋₆ alkyl" and the like.

The term "alkylene", as used herein, alone or in combination with other groups, refers to a saturated straight or branched chain divalent hydrocarbon group. For example, the term "C₁₋₈ alkylene" refers to an alkylene group having 1-8 carbon atoms, such as methylene, ethylene, propylene, butylene, 1-methylethylene, 2-methylethylene or methylpropylene etc.

The term "alkoxy" refers to an alkyl group as defined above attached to an oxygen atom by a single bond. Alkoxy groups are attached to the rest of the molecule through an oxygen atom. An alkoxy group can be represented as -O(alkyl). "C₁₋₈ alkoxy" or "-O(C₁₋₈ alkyl)" refers to an alkoxy group containing 1-8 carbon atoms, wherein the alkyl moiety can be straight chain, branched chain or cyclic structure. Alkoxy groups include, but are not limited to, methoxy, ethoxy, n-propoxy, isopropoxy, n-butoxy, n-pentyloxy, cyclopentyloxy, cyclohexyloxy, and the like.

The term "cycloalkyl" refers to a saturated or unsaturated non-aromatic cyclic hydrocarbon group consisting of carbon atoms and hydrogen atoms, preferably containing 1 or 2 rings. The cycloalkyl may be a monocyclic, fused polycyclic, bridged or spirocyclic structure. The cycloalkyl may have 3-10 carbon atoms, i.e. "C₃₋₁₀ cycloalkyl", such as C₃₋₈ cycloalkyl, C₅ cycloalkyl, C₆ cycloalkyl, C₇ cycloalkyl. Non-limiting examples of cycloalkyl groups include, but are not limited to, cyclopropyl, cyclobutyl, cyclopentyl, cyclohexyl, cycloheptyl, bicyclo[2.2.1]heptyl, spiro[3.3]heptyl, and the like.

The term "heterocyclyl" or "heterocyclic hydrocarbon group" means monocyclic or bicyclic non-aromatic ring systems (three- to ten-membered, three- to eight-membered, three-to six-membered) having, for example, 3-10 (suitably 3-8, more suitably 3-7, 5-7, especially 4-6) ring atoms, wherein at least one ring atom (e.g., 1, 2 or 3) is selected from the group consisting of N, O, and S heteroatoms, and the remaining ring atoms are C. The ring system may be saturated (also understood as the corresponding "heterocycloalkyl") or unsaturated (i.e. having one or more double and/or triple bonds within the ring). The term also covers situations where the C atom may be substituted by oxo (=O) and/or the S atom on the ring may be substituted by 1 or 2 oxo (=O). Heterocyclyl can be, for example, a 4-membered ring such as azetidinyl, oxetanyl; or a 5-membered ring such as tetrahydrofuranyl, dioxanyl, pyrrolidinyl, imidazolidinyl, pyrazolidinyl, pyrrolinyl, oxopyrrolidinyl, 2-oxoimidazolidin-1-yl; or 6-membered rings such as tetrahydropyranyl, piperidinyl, morpholinyl, dithianyl, thiomorpholinyl, piperazinyl, 1,1-dioxo-1,2-thiazinan-2-yl or trithianyl; or a 7-membered ring, such as diazepine ring. Optionally, the heterocyclyl group can be benzo-fused. Heterocyclyl may be bicyclic, without limitation, such as a 5,5-membered ring such as hexahydrocyclopenta[c]pyrrol-2(*1H*)-yl ring; or a 5,6-membered bicyclic ring such as hexahydropyrrolo[1,2-a]pyrazin-2(1H)-yl ring. As mentioned above, the ring containing the nitrogen atom may be partially unsaturated, i.e. it may contain one or more double bonds without limitation, such as 2,5-dihydro-1H-pyrrolyl, 4H-[1,3,4]thiadiazinyl, 4,5-dihydrooxazolyl or 4H-[1,4]thiazinyl ring, or it may be benzo-fused without limitation, such as a dihydroisoquinolinyl ring.

The term "hydrocarbon" solvent refers to a solvent having a straight chain, branched chain or cyclic hydrocarbon having 1-10 carbon atoms. The hydrocarbons may be saturated or unsaturated. Examples of hydrocarbon solvents include, for example, alkane solvents, including but not limited to n-pentane, n-hexane, cyclohexane, n-heptane, octane, or combinations thereof, preferably hexane or heptane. Examples of hydrocarbon solvents also include, for example, aromatic hydrocarbon solvents, each contains at least one aromatic ring and is optionally substituted with straight chain, branched chain or cyclic hydrocarbon group(s). The aromatic hydrocarbon solvents include, but are not limited to, benzene, toluene, xylene or a combination thereof, preferably toluene, xylene or a combination thereof.

The term "haloalkane" solvent refers to the alkane solvents described above, wherein one or more (e.g. 1-6, 1-5, 1-4, 1-3, or 1-2) hydrogen atoms are replaced by halogens. It should be understood by those skilled in the art that when there is more than one halogen substituent, the halogens may be the same or different, and may be located on the same or different C atoms. Halogenated alkane solvents include but are not limited to dichloromethane, trichloromethane, carbon tetrachloride, 1,2-dichloroethane, hexachloroethane and 1,2,3-trichloropropane or combinations thereof, preferably dichloromethane, trichloromethane, 1,2-dichloroethane or combinations thereof, especially dichloromethane.

The term "ester" solvents refer to solvents having esters of 3 to 10 carbon atoms. Ester solvents include, but are not limited to, ethyl acetate, propyl acetate, isopropyl acetate, butyl acetate, amyl acetate or a combination thereof, preferably ethyl acetate, isopropyl acetate, butyl acetate or a combination thereof.

The term "ether" solvents refer to solvents having ethers of 2 to 10 carbon atoms. Examples of ether solvents include, but are not limited to, diethyl ether, isopropyl ether, tetrahydrofuran, methyltetrahydrofuran, dioxane, ethylene glycol dimethyl ether, methyl tert-butyl ether or a combination thereof, preferably tetrahydrofuran, methyltetrahydrofuran, dioxane, ethylene glycol dimethyl ether, methyl tert-butyl ether, or a combination thereof.

The term "nitrile" solvents refer to solvents having nitriles having 2 to 10 carbon atoms. Examples of nitrile solvents include, but are not limited to, acetonitrile, propionitrile, butyronitrile, benzonitrile, phenylacetonitrile, or combinations thereof, preferably acetonitrile, benzonitrile, phenylacetonitrile, or combinations thereof, especially acetonitrile.

The term "disubstituted amide solvents" refer to such amide solvents: an amide formed by linking a C₁₋₃ alkyl acyl group with an amine compound, wherein the amide N atom is substituted with two alkyl groups each independently selected from the group consisting of C₁-3 alkyl groups, or the two substituents on the amide N atom together with the amide N atom to which they are attached form a five to seven membered heterocycle containing one N atom. Examples of disubstituted amide solvents include, but are not limited to, *N,N-*dimethylformamide, *N*,*N*-diethylformamide, *N*,*N*-dimethylacetamide, *N*,*N*-diethylacetamide, N-methylpyrrolidone or a combination thereof.

As used herein, the term "room temperature" refers to about 20-30 °C, preferably about 25 °C.

Unless otherwise stated, percentages, parts, etc. herein are by weight. Unless otherwise stated, the concentration is by weight, the liquid ratio in the mixed solution is calculated by volume, and the ratio (including percentage) of reagents and compounds, and the reaction yield are calculated by molar amount.

The term "crystal form" or "crystal" refers to any solid substance with order in three dimension, which, in contrast to amorphous solid substances, produces a characteristic X-ray powder diffraction pattern with peak(s) having clear boundaries.

The term "X-ray powder diffraction (XRPD) pattern" refers to an experimentally observed diffraction pattern or a parameter, data or value derived therefrom. XRPD patterns are typically characterized by peak position (x-axis) and/or peak intensity (y-axis). XRPD patterns can be measured, for example, on a Bruker D8 advance X instrument.

As used herein, the term "2θ" refers to a peak position expressed in degrees (°) set in the X-ray diffraction experiments, which are generally x-axis unit of a diffraction pattern. If an incident beam is diffracted when it forms an angle theta (θ) with a certain lattice plane, the experimental setting needs to report the reflected beam with an angle 2 theta (2θ). It should be understood that reference herein to specific 2θ values for a specific crystal form is intended to mean the 2θ values (in degrees) as measured using the X-ray diffraction experimental conditions as described herein.

As used herein, the term "substantially" with respect to an X-ray diffraction peak means to take into account representative peak positions and intensity variations. Differences in XRPD patterns between results obtained from separate measurements of the same polymorph may be due to a number of reasons. Sources of error include differences in sample preparation (e.g., sample height), instrumental errors, scaling errors, and operational errors (including errors in determining peak positions). Preferred orientation, that is, the lack of random orientation in the crystallization of the XRPD sample, can lead to significant differences in relative peak heights. Scale errors and sample height errors typically cause all peaks in the diffraction diagram to be displaced by the same amount along the same direction. Often, differences between diffractometers can be compensated for by the same method, resulting in consistence in XRPD peak positions obtained by two different instruments. When these methods are applied to XRPD measurements from the same or different diffractometers, the peak positions for a particular polymorph typically differ by about ±0.2° (2θ). In addition, those skilled in the art will understand that relative peak intensity also varies due to inter-instrument variability as well as the degree of crystallinity, preferred orientation, prepared sample surface, and other factors known to those skilled in the art, and should be considered as a qualitative measure only.

Differential Scanning Calorimetry (DSC) determines the transition temperature when a crystal absorbs or releases heat due to changes in its crystal structure or melting of the crystal. For the same crystal form of the same compound, errors in the thermal transition temperature and melting point are typically within about 5°C in continuous analyses. When describing a compound as having a given DSC peak or melting point, it refers to the said DSC peak or melting point ±5°C. "Substantially" also takes this temperature change into account. DSC provides an auxiliary method for identifying different crystal forms. Different crystal forms can be identified based on their different transition temperature characteristics. It should be noted that the DSC peak or melting point of a mixture may vary over a wide range. In addition, the melting temperature is associated with the rate of temperature rise due to the decomposition during the melting of the substance. DSC patterns can be measured, for example, on a model TA DSC Q200 instrument.

Thermogravimetric analysis (TGA) is a common method for determining the thermal stability of compounds. Herein, TGA is also used to determine the hydration state of a compound. The rate of temperature rise during the test have a certain impact on the pattern. For example, an excessively high rate of temperature rise is not conducive to the detection of intermediate products. TGA patterns can be measured, for example, on a model TA TGA Q500 instrument.

Dynamic Vapor Sorption (DVS) is a common method to investigate the moisture adsorption of drugs, excipients or packaging materials through a dynamically accelerated moisture adsorption process. The moisture adsorption isotherm is usually used to describe the degree of correlation between the moisture content of the sample and the relative humidity during the moisture adsorption process of the sample. DVS patterns can be measured, for example, on a model IGAsorp instrument.

As used herein, "particle size distribution (PSD)" refers to the range of particle size distribution, which can be expressed in terms of the particle size at which the cumulative particle size distribution ratio (e.g., expressed as a fraction, decimal, or percentage) reaches a particular value. For example, D(0.5) or D50 represents the median particle size. The particle size distribution can be measured by a laser light diffraction method, for example, can be measured by a Mastersizer laser particle size analyzer from Malvern Company of the United States.

The term "atom economy" means that in chemical synthesis, synthetic methods and processes are designed for as many as possible incorporation of atoms of the raw materials used in the reaction process into the product molecules. Atom-economical reactions or synthetic routes can increase efficiency and reduce the production of by-products or waste.

The term "pharmaceutically acceptable" means compatibility with the other components of the formulation and without unacceptable toxicity to the subject of administration.

"Pharmaceutically acceptable carrier" refers to those carrier materials which are not significantly irritating to the organism and which do not impair the bioactivity and properties of the active compound. "Pharmaceutically acceptable carriers" include, but are not limited to, glidants, sweeteners, diluents, preservatives, dyes/colorants, flavoring agents, surfactants, wetting agents, dispersing agents, disintegrating agents, stabilizers, solvents or emulsifiers.

The terms "administration" or "administrating" and the like refer to methods by which a compound or composition can be delivered to the desired site of biological action. These methods include, but are not limited to, parenteral (including intravenous, subcutaneous, intraperitoneal, intramuscular, intravascular injection or infusion), topical, rectal administration, and the like.

The term "effective amount" (e.g., "therapeutically effective amount" or "prophylactically effective amount") as used herein refers to the amount of active ingredient which achieves the desired effect to a certain extent upon administration, for example, one or more symptoms of the condition being treated are alleviated or the occurrence of the condition or symptoms thereof is prevented.

"Individual" as used herein includes humans or non-human animals, particularly humans.

The following detailed description of the invention is intended to illustrate non-limiting embodiments, so that other skilled in the art can more fully understand the technical solution, the principle and the practical application of the present invention, so that others skilled in the art may modify and implement the invention in many forms, as are best suited to the requirements of a particular use.

### Preparation method of the present invention

The first aspect of the present invention provides a method for the preparation of a compound of formula (VII), comprising

### Step (A): reacting a compound of formula (VI) with a compound of formula (V) to give a compound of formula (VII)

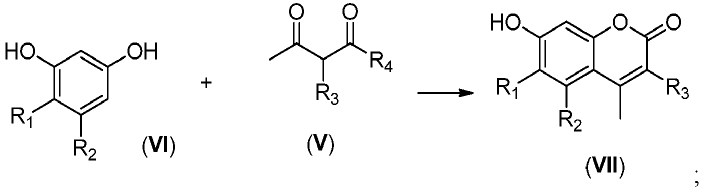

wherein R₁ and R₂ are each independently selected from the group consisting of hydrogen, halogen, and -O(C₁₋₈ alkyl), wherein the alkyl is optionally substituted with 1 or 2 groups selected from the group consisting of halogen, C₁₋₈ alkyl substituted with 1-2 hydroxyl groups, -O(C₁₋₈ alkyl);
R₃ is -L-C(O)NR₅R₆, L is C₁₋₃ alkylene;
R₄ is -O(C₁₋₆ alkyl);
R₅ and R₆ are each independently selected from the group consisting of hydrogen, -O(C₁₋₈ alkyl), C₃₋₁₀ cycloalkyl, wherein the alkyl, cycloalkyl are optionally substituted with 1 or 2 groups selected from the group consisting of halogen, -O(C₁₋₈ alkyl), -NH₂, -NH(C₁₋₈ alkyl), - N(C₁₋₈ alkyl)₂, five- to seven-membered heterocyclyl, wherein the heterocyclyl contains 1-2 heteroatoms selected from the group consisting of N, O, S, and the heterocyclyl is optionally substituted with 1-2 groups selected from the group consisting of C₁₋₈ alkyl; or
R₅ and R₆ together with the N atom to which they are attached form a five- to seven-membered heterocyclyl, and the heterocyclyl optionally contains 1 additional heteroatom selected from the group consisting of N, O, S, and is optionally substituted with 1 or 2 groups selected from the group consisting of halogen, C₁₋₈ alkyl, -O(C₁₋₈ alkyl), -NH₂, -NH(C₁₋₈ alkyl), -N(C₁₋₈ alkyl)₂.

In one embodiment, R₁ and R₂ are each independently selected from the group consisting of hydrogen, halogen, and -O(C₁₋₄ alkyl), wherein the alkyl is optionally substituted with 1 or 2 groups selected from the group consisting of halogen, -O(C₁₋₄ alkyl). In a preferred embodiment, R₁ and R₂ are each independently selected from the group consisting of hydrogen, halogen and -O(C₁₋₂ alkyl), wherein the alkyl is optionally substituted with halogen or methoxy. In a more preferred embodiment, R₁ and R₂ are each independently hydrogen, methoxy or 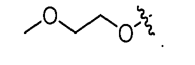 .

In one embodiment, R₄ is -O(C₁₋₄ alkyl), preferably -O(C₁₋₃ alkyl). In a more preferred embodiment, R₄ is -O(C₁₋₂ alkyl). In a particular embodiment, R₄ is ethoxy.

In one embodiment, L is methylene or ethylene. In a preferred embodiment, L is methylene.

In one embodiment, R₅ and R₆ are each independently selected from the group consisting of hydrogen, C₁₋₆ alkyl, wherein the alkyl is optionally substituted with 1-2 groups selected from the group consisting of halogen, -O(C₁₋₆ alkyl), -NH₂, -NH(C₁₋₆ alkyl), -N(C₁₋₆ alkyl)₂, five- to seven-membered heterocyclyl, wherein the heterocyclyl contains 1-2 heteroatoms selected from the group consisting of N, O, S, and the heterocyclyl is optionally substituted with 1-2 groups selected from the group consisting of C₁₋₆ alkyl; or
R₅ and R₆ together with the N atom to which they are attached form a five- to seven-membered heterocyclyl, and the heterocyclyl optionally contains 1 additional heteroatom selected from the group consisting of N, O, S, and is optionally substituted with 1-2 groups selected from the group consisting of halogen, C₁₋₆ alkyl, -O(C₁₋₆ alkyl), -NH₂, -NH(C₁₋₆ alkyl), -N(C₁₋₆ alkyl)₂.

In a preferred embodiment, R₅ and R₆ are each independently selected from the group consisting of hydrogen, C₁₋₄ alkyl, wherein the alkyl is optionally substituted with 1-2 groups selected from the group consisting of halogen, -O(C₁₋₂ alkyl), -NH(C₁₋₂ alkyl), -N(C₁₋₂ alkyl)₂, five- to seven-membered heterocyclyl, wherein the heterocyclyl contains 1-2 heteroatoms selected from the group consisting of N, O, S, and at least one of the heteroatoms is N, and the heterocyclyl is optionally substituted with 1-2 groups selected from the group consisting of C₁₋₄ alkyl; or
R₅ and R₆ together with the N atom to which they are attached form a five- to seven-membered heterocyclyl, and the heterocyclyl optionally contains 1 additional heteroatom selected from the group consisting of N, O, S, and is optionally substituted with 1-2 groups selected from the group consisting of halogen, C₁₋₄ alkyl, -O(C₁₋₂ alkyl), -NH(C₁₋₂ alkyl), -N(C₁₋₂ alkyl)₂.

In a more preferred embodiment, R₅ and R₆ are each independently selected from the group consisting of hydrogen, C₁₋₂ alkyl, wherein the alkyl is optionally substituted with 1-2 groups selected from the group consisting of methoxy, dimethylamino, morpholinyl, piperidinyl or piperazinyl, wherein the morpholinyl, piperidinyl or piperazinyl is optionally substituted with methyl; or
R₅ and R₆ together with the N atom to which they are attached form a morpholinyl, piperidinyl or piperazinyl, wherein the morpholinyl, piperidinyl or piperazinyl is optionally substituted with methyl.

In a further preferred embodiment, R₅ and R₆ are each independently selected from the group consisting of hydrogen, C₁₋₂ alkyl, wherein the alkyl is substituted with morpholinyl; or R₅ and R₆ together with the N atom to which they are attached form a morpholinyl group.

In one embodiment, the compound of formula (VII) has the following structure of compound 5A2, and step (A) is as follows: reacting compound 1 with compound B1 to give compound 5A2

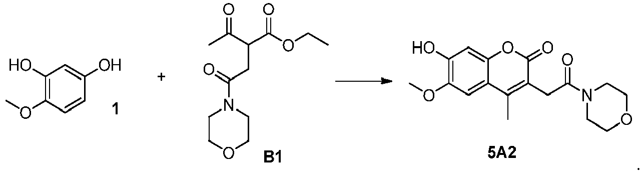

In one embodiment, step (A) is carried out under an acidic condition. In another embodiment, the reaction temperature is -20 °C to 70 °C. In a preferred embodiment, the reaction temperature is -5 °C to 60 °C. In a more preferred embodiment, the reaction temperature is 20 °C to 50 °C.

The second aspect of the present invention provides a method for the preparation of a compound of formula (I), comprising step (A) described in the first aspect of the present invention and the following step (B):

### Step (B): preparing the compound of formula (I) from the compound of formula (VII)

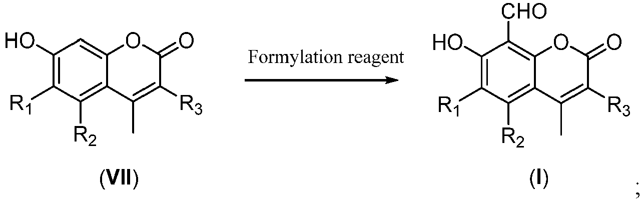

wherein R₁, R₂ and R₃ are as defined in the first aspect of the present invention.

In one embodiment, the formylation reagent is selected from the group consisting of hexamethylenetetramine, paraformaldehyde. In a preferred embodiment, step (B) is carried out in the presence of an acid. In another preferred embodiment, the reaction temperature is 50 °C to 120 °C, preferably 60 °C to 100 °C, more preferably 80 °C to 90 °C.

In one embodiment, after the reaction of step (A) is completed, the compound of formula (I) of the reaction product of step (A) is purified to as follows:
Step i: Mixing the crude compound of formula (I) with halogenated alkane solvent and water to obtain a mixture.
Step ii: Separating the organic phase of the mixture obtained in step i, concentrating to obtain a solid, and rinsing the solid with a suitable solvent to obtain the compound of formula (I) in crystalline state.

In another embodiment, between step i and step ii, it further comprises a step of washing the mixture. In another embodiment, an aqueous alkaline solution is used to wash the mixture.

In yet another embodiment, between step i and step ii, it further comprises a step of adjusting the pH of the mixture, wherein the pH of the mixture is adjusted to near neutrality. In another embodiment, the pH is adjusted to about 6-8, preferably about 7.

In another embodiment, after separating the organic phase and before concentrating, step ii further comprises the step of filtration or centrifugation, and the solution obtained by filtration or centrifugation is concentrated to obtain a solid. Preferably, the step of filtering or centrifuging is filtering. In yet another embodiment, after separating the organic phase, step ii further comprises a step of drying to substantially remove or partially remove moisture in the organic phase.

In another embodiment, the suitable solvent of step ii is an organic solvent. In one embodiment, the organic solvent is selected from the group consisting of nitrile solvents, ester solvents and a combination thereof, preferably nitrile solvents or ester solvents. In one embodiment, the nitrile solvent is selected from the group consisting of acetonitrile, benzonitrile, phenylacetonitrile or a combination thereof, preferably acetonitrile. In another embodiment, the ester solvent is selected from the group consisting of ethyl acetate, isopropyl acetate, butyl acetate or a combination thereof, preferably ethyl acetate.

In another embodiment, the crude compound of formula (I) in step i refers to the residue obtained by concentrating the reaction mixture under reduced pressure to remove the solvent thereof after completion of the reaction in step (A). In yet another embodiment, the crude compound of formula (I) described in step i refers to the residue obtained by: concentrating the reaction mixture after the reaction in step (A) is completed under reduced pressure to gain a residue, dissolving the residue, conducting filtration through a short pad of silica gel, and then concentrating the filtrate.

In one embodiment, after compound of formula (VII) as an intermediate in step (B) is reacted with a formylation reagent to obtain the compound of formula (I), purification is performed according to a method comprising the following steps:
Step i: concentrating the reaction solution, adding water and the compound seed crystal of formula (I) to induce crystallization;
Step ii: separating the crude product obtained in step i, stirring with a suitable solvent under heating conditions, then cooling, and isolating the compound of formula (I) in a crystalline state.

In one embodiment, after separating the crude product and before stirring with a suitable solvent under heating conditions, step ii further comprises a step of rinsing the crude product with a suitable solvent.

In yet another embodiment, after isolating the compound of formula (I) in crystalline state, step ii further comprises a step of rinsing with a suitable solvent and drying.

In another embodiment, the suitable solvent of step ii is selected from the group consisting of nitrile solvents or mixed solvents of nitrile solvents and water. In one embodiment, the nitrile solvent is selected from the group consisting of acetonitrile, benzonitrile, phenylacetonitrile or a combination thereof, preferably acetonitrile.

In another embodiment, the heating temperature in step ii is preferably 40-80 °C, more preferably 50-70 °C.

In another embodiment, the heating time of step ii is at least 2h, preferably at least 4h, more preferably at least 8h.

In one embodiment, the compound of formula (I) has the following structure of compound Orin1001, and step (A) is as follows: reacting compound 5A2 with a formylation reagent to give compound Orin1001

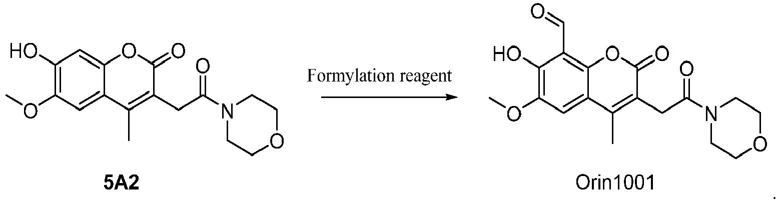

In one embodiment, before step (A), the method of the present invention further comprises the following step (a) and step (b).

### Step (a): Condensing the compound of formula (II) with an amine compound HNR₅R₆ to give a compound of formula (III)

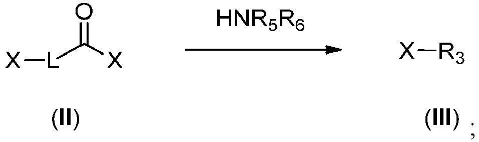

wherein X is halogen; L, R₃, R₅ and R₆ are as defined above. In one embodiment, the reaction is carried out in an organic solvent. In a preferred embodiment, the organic solvent is a neutral aprotic solvent. In a further preferred embodiment, the organic solvent is selected from the group consisting of hydrocarbon solvents, halogenated hydrocarbon solvents, ether solvents, disubstituted amide solvents, ester solvents, and a combination thereof. In one embodiment, the hydrocarbon solvent is selected from the group consisting of toluene, xylene, and a combination thereof. In one embodiment, the halogenated hydrocarbon solvent is dichloromethane. In another embodiment, the ether solvent is selected from the group consisting of tetrahydrofuran, methyltetrahydrofuran, dioxane, ethylene glycol dimethyl ether, methyl tert-butyl ether, and a combination thereof. In yet another embodiment, the disubstituted amide solvent is selected from the group consisting of *N*,*N*-dimethylformamide, *N*,*N*-diethylformamide, *N,N-*dimethylacetamide, *N*,*N*-diethylacetamide, N-methylpyrrolidone, and a combination thereof. In another embodiment, the ester solvent is selected from the group consisting of ethyl acetate, isopropyl acetate, butyl acetate, and a combination thereof. In one embodiment, the reaction temperature is -10 °C to 60 °C, preferably 0 °C to room temperature, more preferably 0 °C to 10 °C.

### Step (b): reacting the compound of formula (III) with the compound of formula (IV) to give a compound of formula (V)

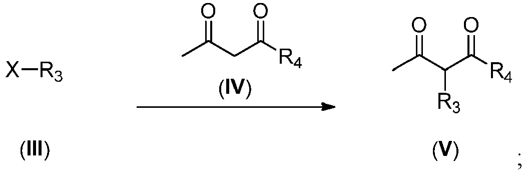

wherein X is halogen; R₃ and R₄ are as defined above. In yet another embodiment, the reaction is carried out in the presence of a base, wherein the base is an organic base or an inorganic base. In one embodiment, the organic base is selected from the group consisting of NaNH₂, sodium alcoholate, K-HMDS, Li-HMDS. In another embodiment, the inorganic base is selected from the group consisting of carbonates and metal hydrides. In one embodiment, the carbonate is selected from the group consisting of sodium carbonate, potassium carbonate, lithium carbonate; preferably sodium carbonate or potassium carbonate. In a particular embodiment, the carbonate is potassium carbonate. In another embodiment, the metal hydride is selected from the group consisting of NaH, KH, LiH, CaH₂. In a particular embodiment, the metal hydride is NaH. In one embodiment, the reaction is carried out in an organic solvent. In a preferred embodiment, the organic solvent is a neutral aprotic solvent. In a further preferred embodiment, the organic solvent is selected from the group consisting of hydrocarbon solvents, halogenated hydrocarbon solvents, ether solvents, disubstituted amide solvents and a combination thereof. In one embodiment, the hydrocarbon solvent is selected from the group consisting of toluene, xylene, and a combination thereof. In one embodiment, the halogenated hydrocarbon solvent is dichloromethane. In another embodiment, the ether solvent is selected from the group consisting of tetrahydrofuran, methyltetrahydrofuran, dioxane, ethylene glycol dimethyl ether, methyl tert-butyl ether, and a combination thereof. In yet another embodiment, the disubstituted amide solvent is selected from the group consisting of *N,N-*dimethylformamide, *N*,*N*-diethylformamide, *N*,*N*-dimethylacetamide, *N*,*N*-diethylacetamide, N-methylpyrrolidone, and a combination thereof. In one embodiment, the reaction temperature is -20 to 100 °C, preferably -20 to 80 °C, more preferably -10 to 60 °C.

In one embodiment, the compound of formula (II) has the following structure of compound D1, the compound of formula (III) has the following structure of compound C1, and step (a) is as follows:

### Step (a): Condensing compound D1 with morpholine to give compound C1

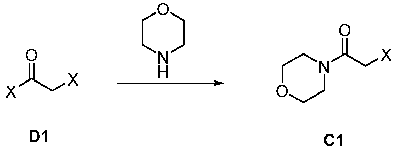

wherein X is halogen.

In another embodiment, the compound of formula (III) has the structure of compound C1, the compound of formula (IV) is ethyl acetoacetate, and the compound of formula (V) has the structure of compound B1, and step (b) is as follows:

### Step (b): reacting compound C1 with ethyl acetoacetate to give compound B1

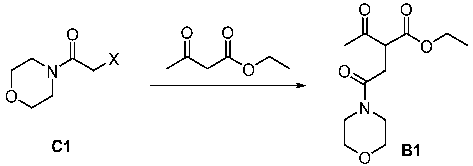

wherein X is halogen.

In one embodiment, X in step (a) or step (b) is selected from the group consisting of Cl and Br. In a preferred embodiment, X is Br.

In one embodiment, the method of the first aspect of the present invention is carried out by the following route: 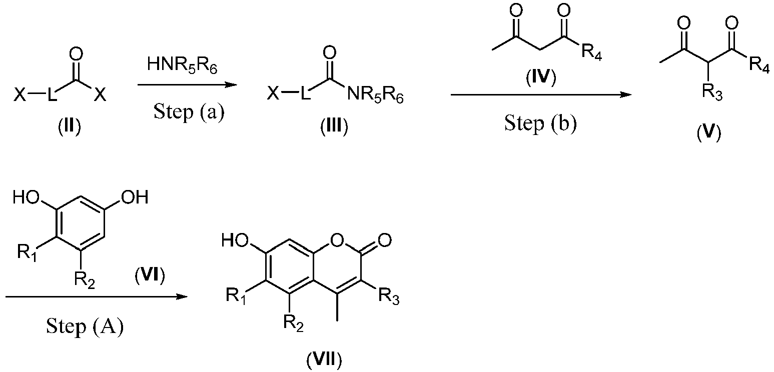 wherein X, L, R₁, R₂, R₃, R₄, R₅, R₆, step (a), step (b) and step (A) are as defined above.

In one embodiment, the method of the second aspect of the invention is carried out by the following route: 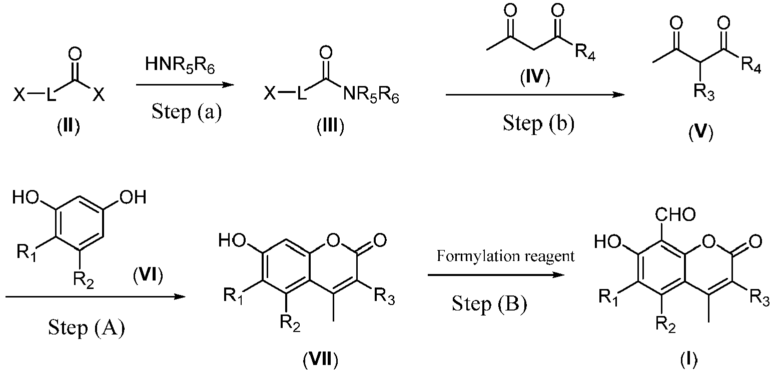 wherein X, L, R₁, R₂, R₃, R₄, R₅, R₆, the formylating reagent, step (a), step (b), step (A) and step (B) are as defined above.

The third aspect of the present invention provides an intermediate compound, and the structure is shown in the following formula (V): 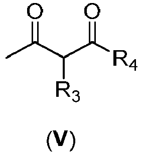 wherein R₃ and R₄ are as defined in the first aspect of the present invention.

In one embodiment,
R₃ is -L-C(O)NR₅R₆, L is methylene or ethylene;
R₄ is -O(C₁₋₆ alkyl);
R₅ and R₆ are each independently selected from the group consisting of hydrogen, C₁₋₆ alkyl, wherein the alkyl is optionally substituted with 1-2 groups selected from the group consisting of halogen, -O(C₁₋₆ alkyl), -NH₂, -NH(C₁₋₆ alkyl), -N(C₁₋₆ alkyl)₂, five- to seven-membered heterocyclyl, wherein the heterocyclyl contains 1-2 heteroatoms selected from the group consisting of N, O, S, and the heterocyclyl is optionally substituted with 1-2 groups selected from the group consisting of C₁₋₆ alkyl; or
R₅ and R₆ together with the N atom to which they are attached form a five- to seven-membered heterocyclyl, and the heterocyclyl optionally contains 1 additional heteroatom selected from the group consisting of N, O, S, and is optionally substituted with 1-2 groups selected from the group consisting of halogen, C₁₋₆ alkyl, -O(C₁₋₆ alkyl), -NH₂, -NH(C₁₋₆ alkyl), -N(C₁₋₆ alkyl)₂.

In another embodiment,
R₃ is -L-C(O)NR₅R₆, L is methylene;
R₄ is -O(C₁₋₆ alkyl);
R₅ and R₆ are each independently selected from the group consisting of hydrogen, C₁₋₄ alkyl, wherein the alkyl is optionally substituted with 1-2 groups selected from the group consisting of the group consisting of halogen, -O(C₁₋₂ alkyl), -NH(C₁₋₂ alkyl), -N(C₁₋₂ alkyl)₂, five- to seven-membered heterocyclyl, wherein the heterocyclyl contains 1-2 heteroatoms selected from the group consisting of N, O, S, and at least one of the heteroatoms is N, and the heterocyclyl is optionally substituted with 1-2 groups selected from the group consisting of C₁₋₄ alkyl; or
R₅ and R₆ together with the N atom to which they are attached form a five- to seven-membered heterocyclyl, and the heterocyclyl optionally contains 1 additional heteroatom selected from the group consisting of N, O, S, and is optionally substituted with 1-2 groups selected from the group consisting of halogen, C₁₋₄ alkyl, -O(C₁₋₂ alkyl), -NH₂, -NH(C₁₋₂ alkyl), -N(C₁₋₂ alkyl)₂.

In yet another embodiment,
R₃ is -L-C(O)NR₅R₆, L is methylene;
R₄ is -O(C₁₋₆ alkyl);
R₅ and R₆ together with the N atom to which they are attached form a morpholine ring. In a particular embodiment, the intermediate compound of formula (V) is compound B1:

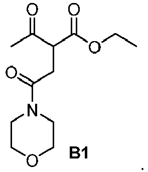

### Crystal form I of the present invention

The fourth aspect of the present invention provides a crystal form I of the compound Orin1001, wherein the X-ray powder diffraction pattern of the crystal form I has characteristic peaks at diffraction angles (2θ) of about 8.44±0.2°, 13.11±0.2°, 15.70±0.2°, 19.73±0.2°, 21.00±0.2° and 22.91±0.2°.

In one embodiment, the X-ray powder diffraction pattern of the crystal form I has characteristic peaks at diffraction angles (2θ) of about 8.44±0.2°, 10.91±0.2°, 10.68±0.2°, 13.11±0.2°, 15.70±0.2°, 17.54±0.2°, 19.73±0.2°, 21.00±0.2°, 22.91±0.2° and 26.27±0.2°. In a preferred embodiment, the X-ray powder diffraction pattern of the crystal form I has characteristic peaks at diffraction angles (2θ) of about 8.44±0.2°, 10.91±0.2°, 10.68±0.2°, 13.11±0.2°, 15.70±0.2°, 16.90±0.2°, 17.54±0.2°, 19.73±0.2°, 21.00±0.2°, 22.91±0.2°, 26.27±0.2°, 28.64±0.2°.

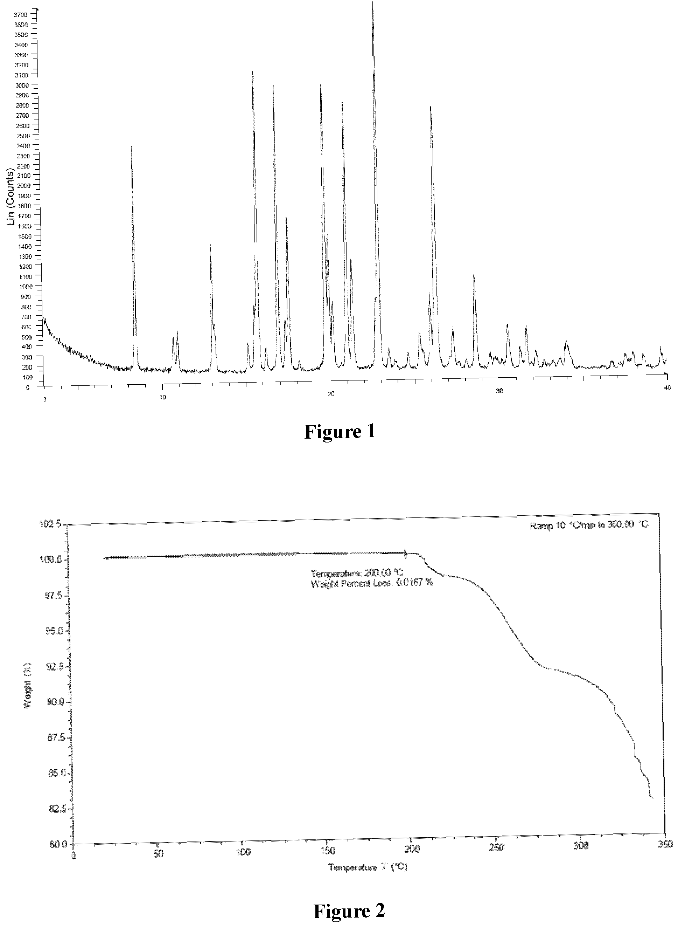

In one embodiment, the X-ray powder diffraction pattern of the crystal form I has characteristic peaks at diffraction angles (2θ) as shown in Table 1. In another embodiment, the X-ray powder diffraction pattern of the crystal form I is substantially as shown in Figure 1.

**Table 1 Orin1001 XRPD 2θ angle data sheet**

| 2θ angle (°) | intensity% | 2θ angle (°) | intensity% |
|---|---|---|---|
| 8.44 | 27.6 | 26.27 | 81 |
| 10.68 | 15.1 | 27.28 | 13.2 |
| 10.91 | 17.2 | 28.06 | 7.1 |
| 13.11 | 23.1 | 28.64 | 30.6 |
| 15.12 | 13.4 | 29.50 | 8.8 |
| 15.70 | 87.7 | 29.79 | 8 |
| 16.19 | 12.3 | 30.58 | 17.9 |
| 16.9 | 36.6 | 31.32 | 9.3 |
| 17.30 | 19.8 | 31.67 | 17.9 |
| 17.54 | 46 | 32.23 | 9.5 |
| 18.15 | 7.5 | 32.70 | 6.3 |
| 19.73 | 87.1 | 33.20 | 6.9 |
| 20.14 | 30.8 | 33.62 | 7 |
| 21.00 | 76.5 | 34.05 | 10.1 |
| 21.34 | 31.7 | 36.21 | 4.3 |
| 22.91 | 100 | 36.73 | 6.2 |
| 23.49 | 9.6 | 37.58 | 8.3 |
| 23.86 | 7.3 | 37.98 | 8.1 |
| 24.61 | 8.3 | 38.63 | 8.5 |
| 25.33 | 17.1 | 39.67 | 6.1 |
| 25.96 | 25.8 | - | - |

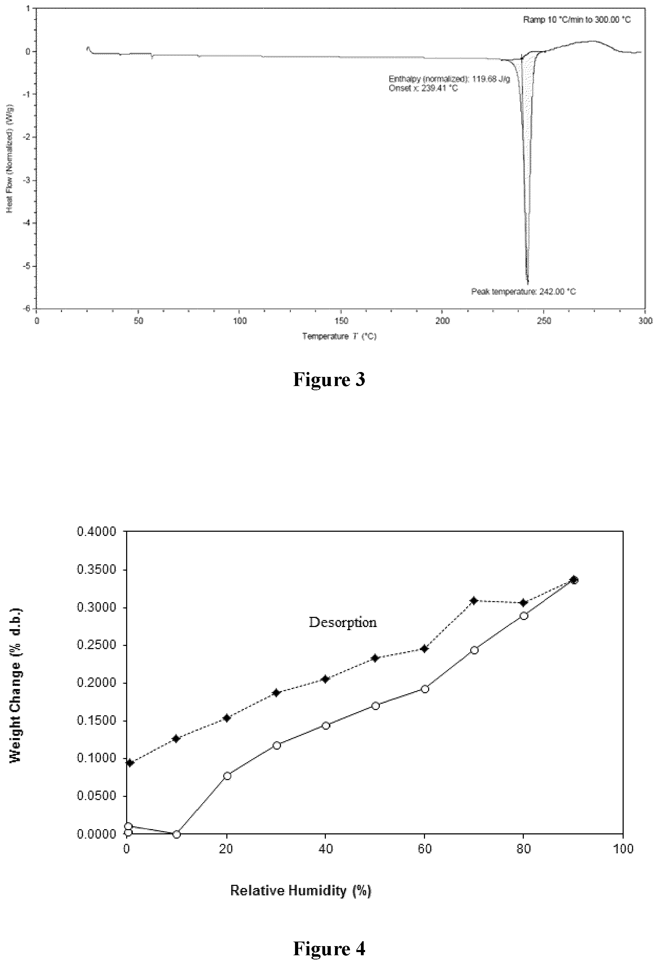

In one embodiment, the crystal form I satisfies at least one of the following (1) to (3):
(1) The thermogravimetric analysis pattern is substantially as shown in Figure 2;
(2) The differential scanning calorimetry analysis pattern is substantially as shown in Figure 3;
(3) The dynamic vapor sorption pattern is substantially as shown in Figure 4.

In one embodiment, the crystal form I has an onset temperature of about 239°C ± 5°C and a peak temperature of about 242°C ± 5°C when characterized by DSC.

In one embodiment, crystal form I is an anhydrate crystal form.

In one embodiment, the X-ray powder diffraction pattern of the crystal form I is determined by the following method: X-ray powder diffractometer: Bruker D8 advance X; radiation source: Cu-Kα; scanning range: 3° (2θ) ~ 40° (2θ); scanning step: 0.02° (2θ); scanning rate: 0.3 sec/step.

In one embodiment, the thermogravimetric analysis pattern of the crystal form I is determined by the following method: TGA thermogravimetric analyzer: TA TGA Q500; temperature range: room temperature ∼ 350 °C; scanning rate: 10 °C/min; protective gas: nitrogen; flow rate: 40 mL/min (balance) or 60 mL/min (sample).

In one embodiment, the differential scanning calorimetry pattern of the crystal form I is determined by the following method: DSC Differential Scanning Calorimeter: TA DSC Q200; temperature range: 25 °C ~ 300 °C; scanning rate: 10 °C/min; protective gas: nitrogen; flow rate: 50 mL/min.

In one embodiment, the dynamic vapor sorption pattern of the crystal form I is determined by the following method: DVS dynamic vapor sorption instrument: IGAsorp; temperature: 25 °C; temperature stability: 0.1 °C/min; carrier gas: nitrogen; flow rate: 250 mL/min; scanning: 2; minimum time: 30 min; maximum time: 2 hours; upper waiting limit: 98%; humidity gradient: adsorption: 0, 10, 20, 30, 40, 50, 60, 70, 80, 90; desorption: 80, 70, 60, 50, 40, 30, 20, 10, 0.

In one embodiment, the particle size distribution (PSD) of the crystal form I is determined using a Malvern Mastersizer 2000. The parameters used may be: pump speed: 2500 rpm; dispersant volume: 800 mL; dispersant: water; dispersion medium: 1% Tween 80.

In one embodiment, the stability of the crystal form I against mechanical treatment is determined by the following method: the crystal form I is placed in a mortar, ground for 2 min and 5 min respectively, and XRPD detection is carried out to analyze the solid after grinding.

In one embodiment, the particle size distribution (PSD) of the crystal form I is shown in Table 2.

**Table 2 Particle size distribution (PSD) data of the crystal form I**

| **D(0.1)** | **D(0.5)** | **D(0.9)** |
|---|---|---|
| 67.1 µm | 115.5 µm | 206.4 µm |

In one embodiment, crystal form I has a bulk density of 0.72 g/ml, a tap density of 0.90 g/ml, a Carr index of 20%, and an angle of repose of 30.6°.

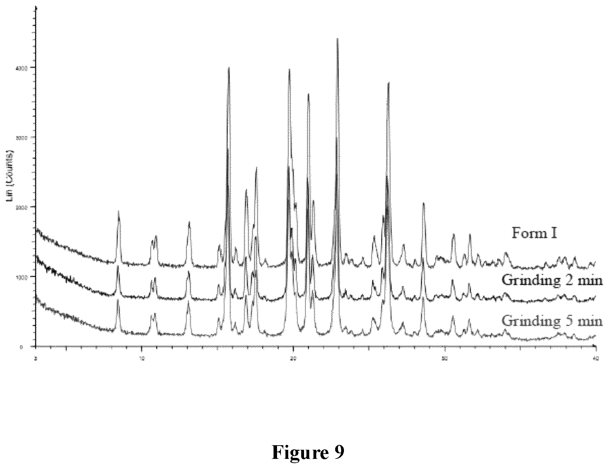

In one embodiment, the result of stability of crystal form I against mechanical treatment is shown in Figure 9.

The crystal form I of the present invention has excellent stability. For example, after mechanical treatment, the diffraction pattern remains substantially unchanged.

### Pharmaceutical compositions and uses

In one aspect, the present invention provides a pharmaceutical composition comprising the crystal form I of the present invention and one or more pharmaceutically acceptable carriers.

In yet another aspect, the present invention also provides the crystal form I of the present invention or the pharmaceutical composition of the present invention in the manufacture of a medicament for the treatment or prevention of a disease, disorder or condition associated with the unfolded protein response (UPR), or the use in the manufacture of a medicament for the treatment or prevention of a disease, disorder or condition associated with a target of regulated IRE1-dependent decay (RIDD).

Yet another aspect of the present invention also relates to the crystal form I of the present invention or the pharmaceutical composition of the present invention for use in the treatment or prevention of a disease, disorder or condition associated with an unfolded protein response, or for use in the treatment or prevention of a disease, disorder or condition associated with a target of regulated IRE1-dependent decay.

Yet another aspect of the present invention also provides a method for treating or preventing a disease, disorder or condition associated with an unfolded protein response, or a method for treating or preventing a disease, disorder or condition associated with a target of regulated IRE1-dependent decay. The method comprises administering to an individual in need thereof an effective amount of the crystal form I of the present invention or a pharmaceutical composition of the present invention, or, bringing a therapeutically effective amount of a crystal form I of the invention or a pharmaceutical composition of the invention for the disease, disorder or condition into a subject.

In one embodiment, the disease, disorder or condition associated with an unfolded protein response is selected from the group consisting of tumors, metabolic-related diseases, immune-related diseases, viral infections, and cardiovascular diseases.

In one embodiment, the disease, disorder or condition associated with an unfolded protein response is selected from the group consisting of B cell autoimmune disease, cancer and viral infection. In one embodiment, the treatable B cell autoimmune disease is selected from the group consisting of the group consisting of: Addison's disease, antiphospholipid syndrome, aplastic anemia, autoimmune hemolytic anemias, autoimmune hepatitis, autoimmune hypophysitis, autoimmune lymphoproliferative disorders, autoimmune myocarditis, Churg-Strauss syndrome, epidermolysis bullosa acquisita, giant cell arteritis, Goodpasture's syndrome, Graves disease, Guillain-Barre syndrome, Hashimoto's thyroiditis, idiopathic thrombocytopenic purpura, IgA nephropathy, myasthenia gravis, pemphigus foliaceous, pemphigus vulgaris, polyarteritis nodosa, polymyositis/dermatomyositis, rheumatoid arthritis, scleroderma, Sjögren's syndrome, systemic lupus erythematosus, Takayasu's arteritis and Wegener's granulomatosis. In one embodiment, the treatable cancer is a solid tumor. In a further embodiment, the solid tumor is selected from the group consisting of breast tumors, bone tumors, prostate tumors, lung tumors, adrenal gland tumors (e.g., adrenocortical tumors), bile duct tumors, bladder tumors, bronchial tumors, nervous tissue tumors (including neuronal and glial tumors), gallbladder tumors, gastric tumors, salivary gland tumors, esophageal tumors, small intestine tumors, cervical tumors, colon tumors, rectal tumors, liver tumors, ovarian tumors, pancreatic tumors, pituitary adenomas and secretory adenomas. In one embodiment, the treatable cancer is a drug-resistant or radiation-resistant solid tumor. In one embodiment, the treatable cancer is a hematological tumor. In a further embodiment, the hematological tumor is a lymphoma or a leukemia. In another embodiment, the hematological tumor is a monoclonal gammopathy of undetermined significance (MGUS), a precursor of myeloma. In one embodiment, the lymphoma is selected from the group consisting of multiple myeloma, Hodgkin's lymphoma, non-Hodgkin's lymphoma (e.g., cutaneous T-cell lymphomas such as Sezary syndrome and mycosis fungoides, diffuse large cell lymphoma, HTLV-1-related T-cell lymphoma, nodular peripheral T-cell lymphoma, extranodal peripheral T cell lymphoma, central nervous system lymphoma, and AIDS-related lymphoma). In yet another embodiment, the leukemia is selected from the group consisting of acute and chronic types of lymphocytic leukemia and acute and chronic types of myeloid leukemias (e.g., acute lymphocytic or lymphoblastic leukemia, acute myelogenous leukemia, acute myeloid leukemia, chronic myelogenous leukemia, chronic lymphocytic leukemia, T-cell prolymphocytic leukemia, adult T-cell leukemia, and hairy cell leukemia). In one embodiment, the treatable viral infection is an enveloped virus infection that utilizes an unfolded protein response pathway when they replicate and form infectious progeny. In a further embodiment, the treatable viral infection is selected from the group consisting of measles virus, pox virus, Ebola virus. In yet another embodiment, the treatable viral infections are those caused by infection with a virus selected from the group consisting of Epstein Barr virus (EBV), cytomegalovirus (CMV), Flaviviruses (e.g., Japanese encephalitis virus and West Nile virus) and hepatitis C virus (HCV).

In one embodiment, the disease, disorder or condition associated with a target of regulated IRE1-dependent decay is selected from the group consisting of neurological disorders, disorders involving overproduction of insulin, and disorders involving inflammation. In a preferred embodiment, the neurological disorder is schizophrenia. In another preferred embodiment, the disorder involving overproduction of insulin is type II diabetes. In yet another preferred embodiment, the disorder involving inflammation is selected from the group consisting of glomerulonephritis, various forms of arthritis, multiple sclerosis and inflammatory bowel disease.

### Beneficial effect

Compared with the methods in the prior art (e.g., CN103079558 A), the method of the present invention has the advantages of simplicity and high efficiency, high atom utilization and high yield.

Intermediate 5A2 was prepared according to the method of CN103079558 A, the route is as follows:

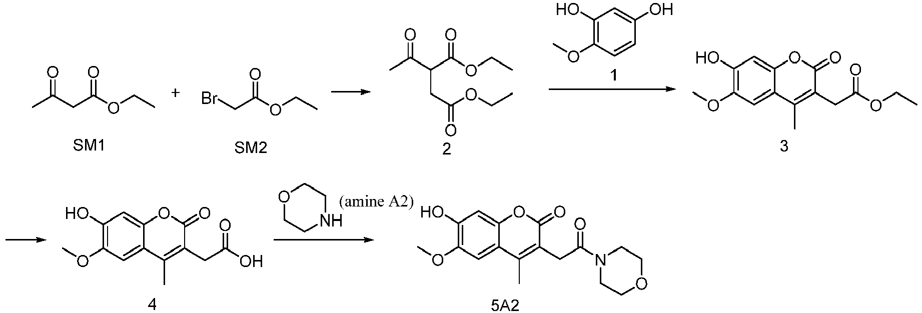

The scheme includes:
Step (1): using ethyl acetoacetate (SM1) and ethyl bromoacetate (SM2) to prepare ethyl acetoacetate compound (compound 2) whose α-position is substituted by ester alkyl group. The yield was 80%. Step (2): preparing a coumarin compound (compound 3) substituted by an ester alkyl group at 3-position. The yield was 60%; Step (3): preparing compound 4. The yield was 67%. Step (4): preparing compound 5A2 by introducing an amide group through acid-amine condensation reaction. Wherein, step (4) was carried out according to the method of Example 62 of CN103079558 A, and crude compound 5A2 was obtained. The yield was 43%. The total yield of the above steps (1) to (4) is about 13.8%, wherein the total yield of the above steps (2) to (4) is about 17.3%.

The above includes a total of 4 steps. Steps (2) to (4) are three-step linear reactions in sequence which are performed in a linear synthesis manner, wherein step (2) constructs a coumarin core with compound 1 through a ring closure reaction, and steps (3) and (4) further introduce an amide group on the 3-position substituent to obtain compound 5A2. In this process, an ester group needs to be introduced into compound 3 first, and then it is removed and converted into an amide group. The steps are cumbersome, with poor atom economy and low yield.

In contrast, in the method for preparing a coumarin compound (e.g., compound 5A2) with 3-position substituted by an amidoalkyl group of the present invention, an alkyl acetoacetate substituted by an amidoalkyl group at the α-position is prepared through the first two steps (see, for example, the methods for the preparation of compounds of formula (V) or for the preparation of the intermediate compound B1, or for example, as shown in Example 1), and then coumarin core was directly constructed through a step of ring closure with compound 1. The total number of the above steps is 3. Through the above-mentioned idea of "building modules first, and then converging", the preparation method of the present invention achieves convergent synthesis, reduces reaction steps in sequence, and is conducive to further improving production efficiency. The inventor unexpectedly found that the utilization of the alkyl acetoacetate compound substituted by amidoalkyl at the α-position to carry out a ring closure reaction with a substituted phenol compound to construct a coumarin core, as in the present invention, is the key for achieving convergent synthesis, reducing reaction steps, and improving the overall reaction yield, atom economy and production efficiency.

In addition, in the preparation method of the present invention, an amide group is introduced through an acyl halide compound, compound of formula (II) (or compound D1), which bears two functional groups. Thus, obviously higher atomic utilization is achieved as compared to the method of introducing ester group, hydrolyzing and then acid amine condensation in CN103079558 A.

The preparation method of the present invention also achieves a higher total yield, which is beneficial to industrialized production. For example, as a representative example of the present invention, the overall yield of Examples 1 to 2 is about 46.6%.

Therefore, the method for preparing a coumarin compound substituted by an amidoalkyl group at 3-position of the present invention has the following advantages: the timing for introduction of the amide group is optimized: the 1,3-dicarbonyl compound comprising amide substitution was prepared first, and then it was used for ring closure. The coumarin core structure and the side chain containing the amide group were efficiently constructed by a facile route, with high utilization rate and high yield, which is conducive to industrial production. In addition, a convergent synthesis route is constructed, which reduces reaction steps in sequence and facilitates further improvement of the production efficiency.

The crystal form I of Orin1001 of the present invention has high purity, high stability, good operability. The preparation method is simple and can be applied to large-scale production.

### Examples

The instruments and equipment used in the following examples and the detection conditions are as follows:
X-ray powder diffractometer: Bruker D8 advance X, the radiation source is Cu-Kα, the scanning range of the test is 3° (2θ) ~ 40° (2θ), the scanning step size is 0.02° (2θ), and the scanning rate is 0.3sec/step.
TGA Thermogravimetric Analyzer: TA TGA Q500, temperature range is from room temperature to 350 °C, scanning rate is 10 °C/min, the protective gas is nitrogen, and the flow rate is 40 mL/min (balance) or 60 mL/min (sample).
DSC Differential Scanning Calorimeter: TA DSC Q200, the temperature range is 25 °C ~ 300 °C, the scanning rate is 10 °C/min, the protective gas is nitrogen, and the flow rate is 50 mL/min.
DVS Dynamic Vapor Sorption Apparatus: IGAsorb, the temperature is 25 °C, the temperature stability: 0.1 °C/min, the carrier gas is nitrogen, the flow rate is 250 mL/min, scanning: 2, the minimum time is 30 min, the maximum time is 2 hours, the upper waiting limit is 98%, humidity gradient: adsorption: 0, 10, 20, 30, 40, 50, 60, 70, 80, 90; desorption: 80, 70, 60, 50, 40, 30, 20, 10, 0.
HPLC (reaction monitoring): Waters Acquity Arc liquid chromatography system or any equivalent; Column: ZORBA×Eclipse×DB C18, 4.6×150 mm, 3.5 µm, or equivalent; mobile phase A: 0.05% trifluoroacetic acid aqueous solution; mobile phase B: 0.05% trifluoroacetic acid acetonitrile solution; concentration gradient running time: 20 minutes.
HPLC (purity determination): Waters Acquity Arc liquid chromatography system or any equivalent; Column: Eclipse×DB C18, 4.6×150 mm, 3.5 µm, or any equivalent; Column temperature: 30°C; detection wavelength: 210 nm; mobile phase A: 0.05% trifluoroacetic acid aqueous solution; mobile phase B: 0.05% trifluoroacetic acid acetonitrile solution; concentration gradient running time: 20 minutes.
LC-MS: Shimadzu LCMS 2010 (column: sepax ODS 50×2.0 mm, 5 µm), or Agilent 1200 HPLC, 1956 MSD (column: Shim-pack XR-ODS 30×3.0 mm, 2.2 µm), or NB-PDM-LCMS-004 (column: Agilen SB-C18 4.6x50mm 1.8 µm, or poroshell SB-C 18 2.7um 3.0^{∗}50mm, SN:USCFE02201), ionization method ES(+).

### Synthetic scheme

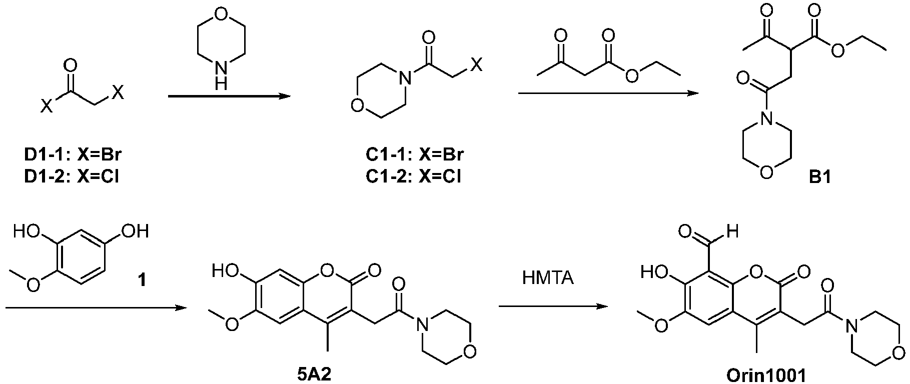

The α-halogenated amide (compound C1) is prepared by first reacting a haloacetyl halide (compound D1) with an amine (morpholine). The α-halogenated amide is then reacted with ethyl acetoacetate to obtain an ethyl acetoacetate compound (the intermediate B1) substituted by an amidoalkyl group at the α-position. Intermediate B1 is subjected to a condensation reaction with a substituted phenol compound (compound 1) and undergoes ring closure to obtain the coumarin compound 5A2 which is substituted by amidoalkyl at 3-position. Compound 5A2 was reacted with hexamethylenetetramine (HMTA) to introduce an aldehyde group to obtain the target compound Orin1001.

### Example 1: Preparation of the intermediate B1

Method A. Preparation of the intermediate B1 using bromoacetyl bromide:

### Step 1: Preparation of Intermediate C1-1

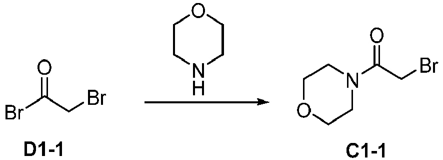

The intermediate C1-1 can be prepared by referring to the method disclosed in Step 1 of Example A9 in International Patent Application WO1999032436, or by the following Method 1 or Method 2.

### Method 1 (low temperature reaction):

Dichloromethane (75.0 L) and bromoacetyl bromide (D1-1, 5.0 kg, 1.00 eq.) was added to the reaction kettle. The temperature was cooled down to 0 °C ~ 5 °C. Morpholine (4.32 kg, 2.00 eq.) pre-cooled to 0 °C ~ 5 °C was added. The reaction was stirred at 0-5 °C for at least 0.5 hours. The reaction was monitored with TLC until the starting material at Rf=0 disappeared. The reaction was quenched with saturated aqueous NH₄Cl (25.0 L) at 0-5 °C and stirring was continued for at least 20 min. The reaction solution was allowed to stand to separate the layers. Then the organic phase was separated and washed with saturated aqueous NH₄Cl solution (25.0 L). The organic phase was concentrated under reduced pressure at ≤45 °C until no obvious fractions dripped out. Another batch was prepared on the same scale. The two batches were combined to obtain a total of 9.25 kg of the intermediate 2-bromo-1-morpholinoethan-1-one (C1-1). The yield was 90.0%, and the purity detected by HPLC was 99.6%. H¹ NMR(300 MHz, DMSO-*d*₆) δ 3.44-3.49 (m, 4H), 3.55-3.69 (m, 4H) 4.13 (S, 2H). LC-MS: [M+1]⁺=208.1

### Method 2 (room temperature reaction):

Under nitrogen protection, bromoacetyl bromide (D1-1, 250 g, 1.00 eq.) and dichloromethane (3.75 L) were added to a 5 L four-neck flask, and the temperature was lowered to 5°C with a brine bath. Morpholine (216g, 2.00eq.) was added dropwise and the temperature was controlled to 0~10 °C. After the dropwise addition, the reaction temperature was controlled to be no more than 10 °C, and the mixture was stirred for 30 minutes. The brine bath was removed, and the reaction was carried out at room temperature for 2 hours. TLC detection showed that bromoacetyl bromide was completely consumed. The temperature of the system was lowed to be no more than 10 °C, and saturated aqueous NH₄Cl solution (1.25 L) was added dropwise. The layers of the mixture were separated and the organic phase was washed once more with saturated aqueous NH₄Cl (1.25 L). The organic phase was concentrated under reduced pressure at ≤45 °C until no obvious fraction dripped out to obtain 234.8 g of the intermediate 2-bromo-1-morpholinoethan-1-one (C1-1). Yield: 90.0%. KF = 0.27%, purity detected by HPLC was 99.2%. H-NMR (DMSO-d6) δ 3.40-3.50 (m, 4H), 3.50-3.60 (m, 4H), 4.11 (s, 2H).

### Step 2: Preparation of the intermediate B1

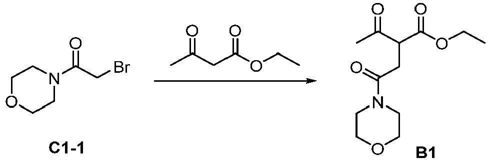

### Method 1 (with NaH as base):

To the intermediate C1-1 (4.93 kg, 1.00 eq.) was added THF (4.45 kg, 5.0 L). Stirring was conducted to obtain a clear solution of the intermediate C1-1 in THF, which was cooled to 0~5 °C for later use. THF (17.8 kg, 20.0 L) was added to the reaction kettle, followed by NaH (60%, 0.845 kg, 1.10 eq.), and the temperature was lowered to 0~5 °C. Ethyl acetoacetate (2.50 kg, 1.00 eq.) pre-cooled to 0~5 °C was added to the kettle, stirred at 0~5 °C for at least 0.5 hours, and then the intermediate C1-1 prepared as above was added in THF at 0~5 °C. Stirring of the reaction was continued to 0~5 °C for at least 2 hours. Samples were sent to HPLC to monitor the reaction until the peak area of the intermediate C1-1 was ≤5.0%. Saturated aqueous NH₄Cl solution (35.0 L) was added at 0~5 °C, and the mixture was allowed to stand to separate the layers. The aqueous phase was extracted with ethyl acetate (25.0 L), the organic layers were combined and concentrated under reduced pressure at about 45 °C to 5.0±1.0 V, water (6.5 L) was added, and the mixture was stirred for 15 minutes. The mixture was allowed to stand to separate the layers, and the organic layer was concentrated under reduced pressure at 45 °C until no obvious fractions dripped. To the residue was added n-heptane (5.0 L), and after stirring for at least 0.5 hours, the mixture was allowed to stand to separate the layers, and the lower layer liquid was collected into a bottle to obtain 4.45 kg of the intermediate ethyl 2-acetyl-4-morpholinyl-4-oxobutanoate (B1). The yield was 90.0% (crude product), wherein the purity of the intermediate B1 detected by HPLC was 84.7%, which was used directly in the next reaction. ¹H NMR :(300MHz, DMSO-*d*₆) δ 1.17-1.24 (m, 3H), 2.27 (s, 3H), 2.77-2.94 (m, 2H), 3.38-3.60 (m, 8H), 3.98-4.05 (m, 1H), 4.07-4.16 (m, 2H). LC-MS: [M+1]⁺=258.2

### Method 2 (with potassium carbonate as the base):

2-1: THF (8.90 kg, 10.0 L), the intermediate C1-1 (1.89 kg, 1.05 eq.) and potassium carbonate (3.19 kg, 3.00 eq.) were added to the reaction kettle, and stirring was conducted for at least 15 hours at 40 ± 5°C, and sample were subjected to HPLC to monitor the reaction until the peak area of the intermediate C1-1 was ≤ 5.0%. The reaction mixture was cooled to room temperature, filtered, and the filter cake was rinsed with THF (2.0 L × 2). The combined filtrates were washed with sodium chloride aqueous solution. The organic layers were combined and concentrated under reduced pressure at about 45°C until no obvious fractions dripped to obtain 1.99 kg of the intermediate ethyl 2-acetyl-4-morpholinyl-4-oxobutanoate (B1). The yield was 101% (crude product), and the purity of the intermediate B1 was 86.2% detected by HPLC, which was directly used in the next reaction. The characterization results of B1 were consistent with the above.

2-2: THF (20.55kg, 10V), the intermediate C1-1 (4.37kg, 1.05 eq.), ethyl acetoacetate (2.32kg, 1.00eq.) and potassium carbonate (7.40kg, 3.00 eq.) were added to the reaction kettle, and stirring was conducted for at least 16 hours at 40 ± 5°C. Samples were subjected to HPLC to monitor the reaction until the peak area of the intermediate C1-1 was ≤ 5.0%. If the reaction is not complete after 16h, a small amount of water was added to the reaction mixture, and stirring was conducted at 40±5°C for 4 h. One repetition was conducted if necessary, until the peak area of the intermediate C1-1 was ≤5.0%. The reaction mixture was cooled to 25±5°C, filtered, and the filter cake was rinsed with THF (2.0 V × 2). The combined filtrates were washed with aqueous solution of sodium chloride. The organic layers were combined and concentrated under reduced pressure at about 45°C until no obvious fractions dripped to obtain 4.87kg of the intermediate ethyl 2-acetyl-4-morpholinyl-4-oxobutanoate (B1). The yield was 106%, and the purity of the intermediate B1 was 86.2% detected by HPLC. The characterization results of B1 were consistent with the above.

### Method B. Preparation of the intermediate B1 using chloroacetyl chloride:

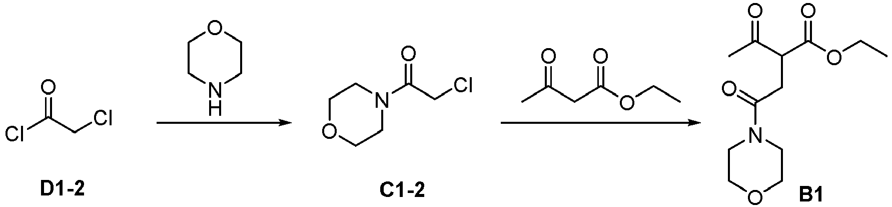

Step 1: Preparation of the intermediate C1-2:
The intermediate C1-2 can be prepared by referring to the method disclosed in Example 57 Part A of US Patent No. 5753660 A, or by the following method.

Under nitrogen protection, dichloromethane (1.54 L) and chloroacetyl chloride (D1-2, 300 g, 1.05 eq.) were added to a 5 L four-neck flask, and cooled to ≤10 °C with an ice-water bath. K₂CO₃ (523g, 1.5 eq.)was added in batches. The temperature was controlled to ≤20 °C_{∘} Morpholine (220g, 1.0 eq.) was added dropwise and the temperature was controlled to ≤20 °C. White mist was generated and exothermic heat was observed. After the dropwise addition. The temperature was allowed to warm to 20 °C. Stirring was conducted for 3h. Detection was conducted using TLC. Chloroacetyl chloride disappeared. The temperature was controlled to ≤10 °C with an ice-water bath. Water was added dropwise. Exothermic heat was observed. The mixture was allowed to stand to separate the aqueous and organic phases, and the aqueous phase was extracted with dichloromethane (1.5 L × 2). The organic phases were combined and washed with water (1.0 L × 2). The organic phase was dried over sodium sulfate. The organic phase was concentrated under reduced pressure until there were no obvious fractions to obtain the intermediate 2-chloro-1-morpholinoethan-1-one (C1-2, 290 g) as a yellow oily liquid. The yield was 70%, and the purity was 97% detected by HPLC. ¹H-NMR(300 MHz, CDCl₃): δ 3.51-3.54 (m, 2H), 3.61-3.64 (m, 2H), 3.68-3.74 (m, 4H), 4.06 (s, 2H). LC-MS: [M+1]⁺=164

### Step 2: Preparation of the intermediate B1:

To a 500 mL three-necked flask was added ethyl acetoacetate (23.86 g, 1.5 eq.) and methyl tert-butyl ether (160 mL). The temperature was cooled to 0 °C, and potassium carbonate (25.34 g, 1.5 eq.) was added. The reaction temperature was controlled to 0-5 °C and stirring was conducted for 30 minutes. A solution of the intermediate C1-2 (20.00 g, 1.0 eq.) in methyl tert-butyl ether was added. The temperature was heated to 50 °C, and the reaction was conducted overnight. Samples are collected and subjected to HPLC detection, which showed that the content of C1-2 was 2.2%. After filtration, the filter cake was rinsed with dichloromethane, and the filtrate was concentrated under reduced pressure to obtain an oil. The oil was dissolved in dichloromethane and washed with water. The aqueous phase was extracted once with dichloromethane. The organic phases were combined and washed with saturated sodium chloride solution. The organic phase was concentrated to dryness under reduced pressure to obtain 39 g of the intermediate ethyl 2-acetyl-4-morpholinyl-4-oxobutanoate (B1) as light yellow oil. The yield was 124.1% (crude product). The purity of the intermediate B1 was 88.6% detected by HPLC, which was directly used in the next reaction.

### Example 2: Preparation of the intermediate 5A2

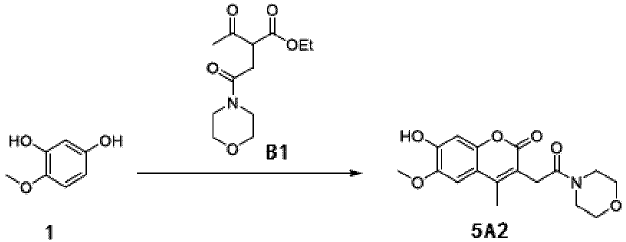

1: To the reaction kettle was added methanesulfonic acid (19.98 kg, 13.5 L). The temperature was controlled to ≤ 40 °C. 4-methoxy-1,3-benzenediol (1, 2.7 kg, 1.00 eq.) was added. Additional Intermediate B1 (5.5 kg, 1.10 eq.) was added. The reaction was stirred at 40 ± 5 °C for at least 18 hours, and samples were subjected to HPLC to monitor the reaction until the peak area of starting material 1 was ≤ 5.0%. The reaction solution was cooled to 0~5 °C. The temperature was controlled to ≤40 °C. Water (27.0 L) was added. The temperature was cooled to 0~5 °C and stirring was conducted for 16 hours. The mixture was centrifuged, and the resulting solid was rinsed with water (8.0 L) and acetonitrile (5.5 L × 2) respectively to give a wet solid. The reaction kettle was purged with nitrogen three times. Acetonitrile (13.5 L) and the wet solid obtained above were added. The temperature was heated to 70±5 °C and stirring was conducted for at least 1 hour. The temperature was cooled to 25±5 °C, the mixture was centrifuged. The solid obtained by centrifugation was rinsed with acetonitrile (4.0 L × 2), then transferred to a vacuum oven. The temperature was controlled to 40 ∼ 45 °C (oven temperature). Vacuum drying was conducted for at least 8 hours. Cooling was conducted to room temperature to obtain 5 g of intermediate 7-hydroxy-6-methoxy-4-methyl-3-(2-morpholino-2-oxoethyl)-2H-benzopyran-2-one (5A2). The yield was 60%. The purity was 99.5%. ¹H-NMR: (300 MHz /DMSO-*d*₆) δ 2.31(s, 3H), 3.44-3.45 (m, 2H) 3.55-3.65 (m, 8H), 3.87(s, 3H), 6.78(s, 1H), 7.16(s, 1H), 10.19(s, 1H). LC-MS:[M+1]⁺=334.2
2: To reaction kettle A was added methanesulfonic acid (50mL, 5.0V). The temperature was controlled to ≤40 °C. The intermediate B1 (25.06g, 0.95 eq.) was added, and then 4-methoxy-1,3-benzenediol (1, 10.0 g, 1.00 eq.) was added and the reaction was stirred at 40 ± 5 °C for at least 24 hours. Samples were sent to HPLC to monitor the reaction until the peak area of the starting material 1 was ≤5.0%. If the reaction was not complete after 24 h and the residual intermediate B1 was less than 4%, the intermediate B1 (0.05eq.) was added dropwise at 40±5 °C, and stirring was conducted for at least 5 hours. The operation was repeated if necessary until the peak area of starting material 1 was ≤5.0%. Water (100 mL, 10.0V) was added to the reaction kettle B, then the reaction mixture of the reaction kettle A was added dropwise to the reaction kettle B, and stirring was conducted at 2.5±2.5°C for at least 16 hours. The mixture was centrifuged, and the resulting solid was rinsed with water (30mL, 3.0V) and acetonitrile (20mL×2 , 2.0 V × 2) respectively to give a wet solid. The reaction kettle A was purged with nitrogen three times. Acetonitrile (50mL, 5.0V) and the wet solid obtained above were added. The temperature was heated to 70±5 °C and stirring was conducted for at least 8 hour. The temperature was cooled to 25-30 °C. The mixture was centrifuged. The solid obtained by centrifugation was rinsed with acetonitrile (15mL×2, 1.5V×2), then transferred to a vacuum oven. The temperature was controlled to 40 ~ 45 °C (oven temperature). Vacuum drying was conducted for at least 8 hours. Cooling was conducted to room temperature to obtain 8.5g of intermediate 7-hydroxy-6-methoxy-4-methyl-3-(2-morpholino-2-oxoethyl)-2H-benzopyran-2-one (5A2). The yield was 36%, purity 99.7%. The characterization results were consistent with the above.

### Example 3: Preparation of compound Orin1001

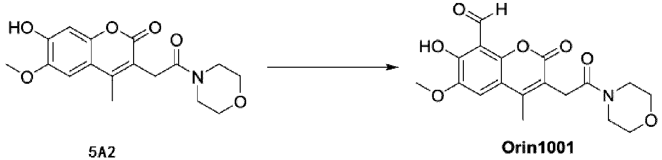

### method 1:

The reaction flask was charged with intermediate 5A2 (10 g, 1.00 eq.), hexamethylenetetramine (HMTA, 16.8 g, 4.00 eq.) and trifluoroacetic acid (500 mL) and heated at 90 °C for 1.5 hours under nitrogen atmosphere. The reaction was complete as detected by LC-MS. The reaction solution was cooled to room temperature and concentrated under reduced pressure to remove the solvent to obtain a residue. To the obtained residue was added water (30 mL). Neutralization was conducted with 10% sodium bicarbonate aqueous solution. Dichloromethane extraction (1000 mL × 3). The organic phases were combined, dried over sodium sulfate, concentrated, and washed with acetonitrile and ethyl acetate respectively to obtain compound Orin1001 (yellow solid, 5 g). The yield was 46%. ¹H NMR (TH03493-010-2M CDCl₃, 400 MHz): δ 12.45 (s, 1H, OH), 10.59 (s, 1H, CHO), 7.24 (s, 1H, ArH), 3.95 (s, 3H, CH₃), 3.78-3.62 (m, 10H, CH₂), 2.46 (s, 3H, CH₃). LC-MS [M+H]⁺ = 362.20_{∘}

X-ray powder diffraction (XRPD) detection was performed on the finally obtained compound Orin1001. It was demonstrated to be in a crystalline state, and was named as crystal form I.

The crystal form I was subjected to TGA, DSC, DVS and stability tests. The results are as follows.

### 1) XRPD detection

The XRPD of the crystal form I is substantially as shown in Figure 1. The 2θ data sheet is shown in Table 1.

### 2) TGA test

According to the thermogravimetric analysis (TGA) results, when heated to 200 °C, there was a weight loss gradient of about 0.017%, and the thermogravimetric analysis pattern is substantially as shown in Figure 2. It can be seen from Figure 2 that Orin1001 crystal form I is anhydrous.

### 3) DSC test

According to the results of differential scanning calorimetry (DSC), an endothermic peak begins to appear when heated to around 239.4 °C, and the differential scanning calorimetry pattern is substantially as shown in Figure 3. It can be seen from Figure 3 that the onset temperature (Tonset) of Orin1001 crystal form I is about 239 °C±5°C, for example, 239 °C; the peak temperature (Tpeak) is about 242 °C±5°C, for example, 242 °C.

### 4) DVS test

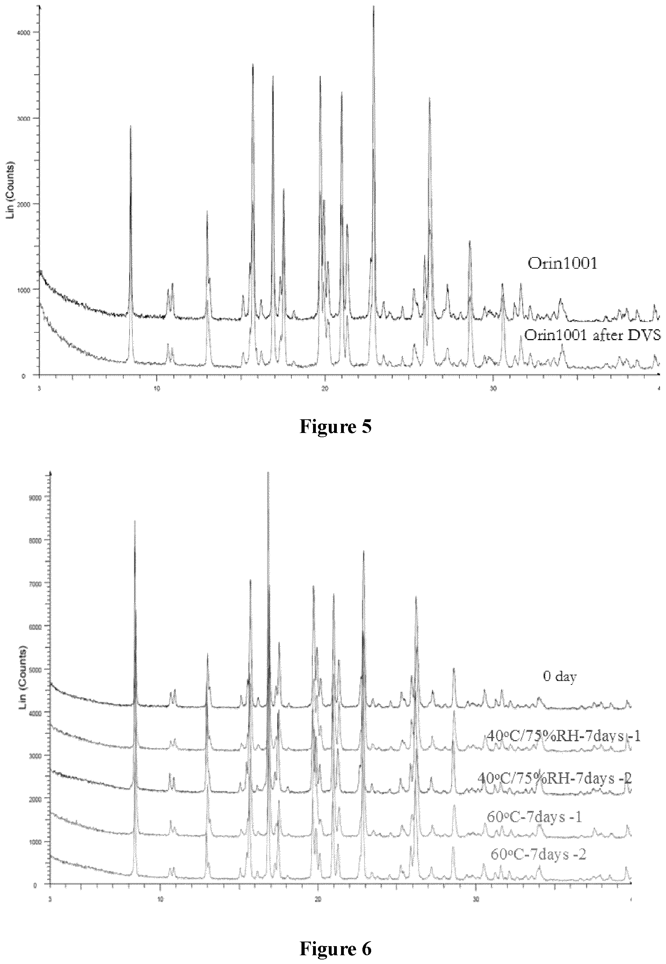

According to the results of dynamic vapor sorption (DVS), the crystal form I gained 0.34% in weight after equilibration at 90% relative humidity, demonstrating only slightly hygroscopic. The crystal form did not change after the DVS measurement. The DVS detection results are substantially as shown in Figure 4, and the XRPD comparison charts before and after DVS detection are substantially as shown in Figure 5. It can be seen from Figure 4 and Figure 5 that the Orin1001 crystal form I is slightly hygroscopic.

### 5) Stability test

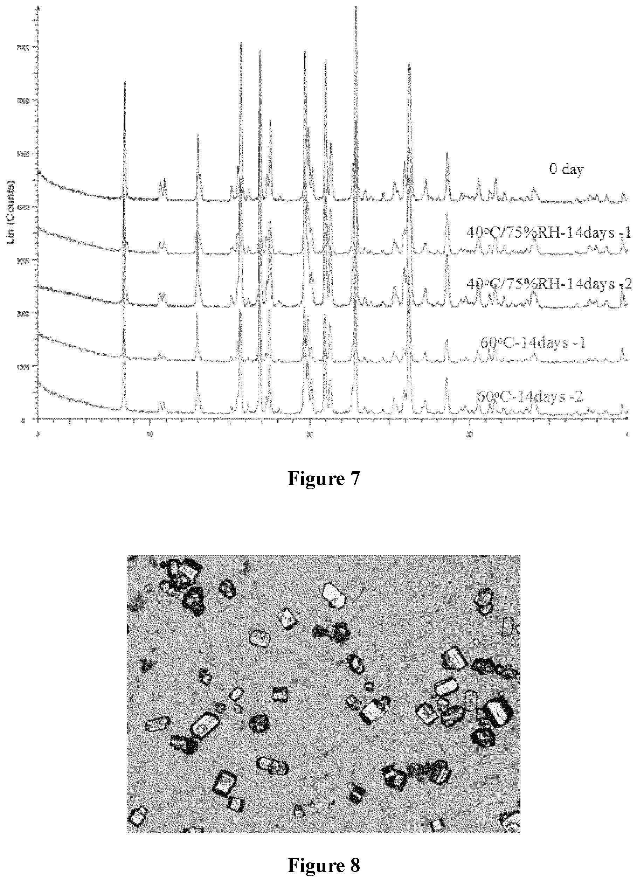

Two samples of the crystal form I were placed under the conditions of 40 °C/75% relative humidity (RH), and the temperature in the oven was 60 °C (with humidity not controlled) for 14 days. Samples were taken on the 0th, 7th and 14th days for HPLC purity detection and XRPD detection, and the results showed that Form I had good chemical and physical stability (Table 3, Table 4, Figure 6, Figure 7).

**Table 3 crystal form I stability test results (HPLC purity: peak area %) - the 7th day**

| | **T0** | **40 °C/75%RH** | **60 °C (in oven)** |
|---|---|---|---|
| **Sample-1** | 99.60 | 99.62 | 99.64 |
| **Sample-2** | 99.58 | 99.54 | 99.58 |

**Table 4 crystal form I stability test results (HPLC purity: peak area %) - the 14th day**

| | **T0** | **40 °C/75%RH** | **60 °C (in oven)** |
|---|---|---|---|
| **Sample-1** | 99.53 | 99.54 | 99.52 |
| **Sample-2** | 99.54 | 99.56 | 99.54 |

It can be seen from Table 3, Table 4, Figure 6 and Figure 7 that Orin1001 crystal form I has excellent stability.

### method 2:

Trifluoroacetic acid (230.40 kg, 150.0 L, 20.0 V) was added to the reaction kettle, and the temperature was lowered to 0 ~ 10 °C. The temperature was controlled below 10°C, and hexamethylenetetramine (HMTA, 23.44 kg, 7.50 eq.) and the intermediate 5A2 (7.44 kg, 1.00 eq.) were added. The temperature was adjusted to 80±5°C and the reaction was stirred for at least 40 hours. Samples were sent to HPLC to monitor the reaction until the peak area of intermediate 5A2 was ≤ 5.0%. The reaction solution was concentrated under reduced pressure below 70°C to about 120 L. The temperature was cooled to 0 ~ 10 °C. Purified water (200.0 L) and Orin1001 seed crystals (1.00 wt%) were added. The temperature was kept at 0~10°C and the reaction was stirred for at least 16 hours. After centrifugation, the filter cake was rinsed with purified water (5.0 L) and acetonitrile (5.0 L × 2). The reaction kettle was purged with nitrogen three times. To the reaction kettle were added acetonitrile (27.0 L), purified water (13.5 L) and the wet filter cake obtained above. The temperature was heated to 60±5°C and the reaction was stirred for at least 8 hours. The temperature was lowered to 25~30°C, centrifuged, and the filter cake was rinsed with acetonitrile (7.5 L × 2). The filter cake was transferred to a vacuum oven. The temperature was controlled at 40 ~ 45 °C (oven temperature). Vacuum drying was conducted for at least 4 hours. The drying was stopped, and the temperature in the oven was lowered to below 30° C to obtain Orin1001 (yellow solid, 4.61 kg) with a yield of 57% and a purity of 99.9% detected by HPLC. The characterization results were consistent with the above.

The crystal form I was tested for particle size distribution (PSD) and stability against mechanical treatment. The results are as follows.

### 1) Particle size distribution (PSD) test of the crystal form I

Crystal form I is a granular crystal, and the particle size is 50-100 µm observed under a microscope (Nikon Eclipse LV100POL).

The particle size distribution (PSD) of the crystal form I was determined using a Malvern Mastersizer 2000. Pump speed: 2500 rpm; Dispersant volume: 800mL; Shielding degree: 10%-20%; Dispersant: water; Dispersion medium: 1% Tween 80; Background measurement: 12s; Sample measurement: 12s; Measurement cycle: 3. The results are as follows:

| **D(0.1)** | **D(0.5)** | **D(0.9)** |
|---|---|---|
| 67.1 µm | 115.5 µm | 206.4 µm |

The bulk density of the crystal form I is 0.72 g/ml, the tap density is 0.90 g/ml, the Carr index is 20%, the angle of repose is 30.6°, and the fluidity is very good. The powder with good fluidity is less likely to raise dust, and has low adsorption and low viscosity, and facilitates industrial application.

### 2) Test of stability against mechanical treatment

The crystal form I was placed in a mortar, ground for 2 min and 5 min, respectively, and XRPD detection was carried out to analyze the solid after grinding. The results are shown in Figure 8. The results show that after grinding for 2 min and 5 min, the crystal form does not change, indicating that the crystal form I has good stability against mechanical treatment, which is conducive to large-scale industrial production.

While typical embodiments of the invention have been illustrated and described, the invention is not limited to the details described. Since various possible modifications and substitutions are possible without departing from the spirit of the invention, those skilled in the art will be able to use routine experimentation to devise modifications and equivalents of the invention, therefore, all such modifications and equivalents are intended to fall within the spirit and scope of the invention as defined by the following claims.

## Claims

1. A method for the preparation of a compound of formula (VII), comprising
Step (A): reacting a compound of formula (VI) with a compound of formula (V) to give a compound of formula (VII) 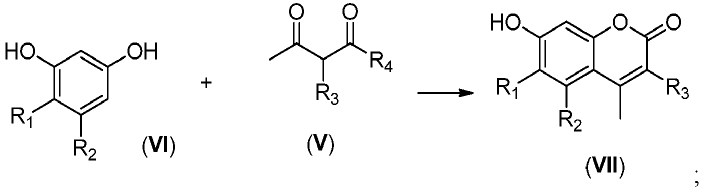 wherein R₁ and R₂ are each independently selected from the group consisting of hydrogen, halogen, and -O(C₁₋₈ alkyl), wherein the alkyl is optionally substituted with 1-2 groups selected from the group consisting of halogen, C₁₋₈ alkyl substituted with 1-2 hydroxyl groups, -O(C₁₋₈ alkyl);
R₃ is -L-C(O)NR₅R₆, L is C₁₋₃ alkylene;
R₄ is -O(C₁₋₆ alkyl);
R₅ and R₆ are each independently selected from the group consisting of hydrogen, C₁₋₈ alkyl, C₃₋₁₀ cycloalkyl, wherein the alkyl, cycloalkyl are optionally substituted with 1-2 groups selected from the group consisting of halogen, -O(C₁₋₈ alkyl), -NH₂, -NH(C₁₋₈ alkyl), -N(C₁₋₈ alkyl)₂, five- to seven-membered heterocyclyl, wherein the heterocyclyl contains 1-2 heteroatoms selected from the group consisting of N, O, S, and the heterocyclyl is optionally substituted with 1-2 groups selected from the group consisting of C₁₋₈ alkyl; or
R₅ and R₆ together with the N atom to which they are attached form a five- to seven-membered heterocyclyl, and the heterocyclyl optionally contains 1 additional heteroatom selected from the group consisting of N, O, S, and is optionally substituted with 1-2 groups selected from the group consisting of halogen, C₁₋₈ alkyl, -O(C₁₋₈ alkyl), -NH₂, -NH(C₁₋₈ alkyl), -N(C₁₋₈ alkyl)₂.

2. The method of claim 1, wherein R₁ and R₂ are each independently selected from the group consisting of hydrogen, halogen and -O(C₁₋₄ alkyl), wherein the alkyl group is optionally substituted with 1-2 groups selected from the group consisting of halogen, -O(C₁₋₄alkyl); preferably, R₁ and R₂ are each independently selected from the group consisting of hydrogen, halogen and -O(C₁₋₂ alkyl), wherein the alkyl is optionally substituted with halogen or methoxy; more preferably, R₁ and R₂ are each independently hydrogen, methoxy or

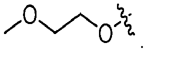

3. The method of claim 1, wherein R₄ is -O(C₁₋₄ alkyl); -O(C₁₋₂ alkyl); more preferably ethoxy.

4. The method of claim 1, wherein L is methylene or ethylene, preferably methylene.

5. The method of claim 1, wherein R₅ and R₆ are each independently selected from the group consisting of hydrogen, C₁₋₆ alkyl, wherein the alkyl is optionally substituted with 1-2 groups selected from the group consisting of halogen, -O(C₁₋₆ alkyl), -NH₂, -NH(C₁₋₆ alkyl), - N(C₁₋₆ alkyl)₂, five- to seven-membered heterocyclyl, wherein the heterocyclyl contains 1-2 heteroatoms selected from N, O, S, and the heterocyclyl is optionally substituted with 1-2 groups selected from the group consisting of C₁₋₆ alkyl; or
R₅ and R₆ together with the N atom to which they are attached form a five- to seven-membered heterocyclyl, and the heterocyclyl optionally contains 1 additional heteroatom selected from the group consisting of N, O, S, and is optionally substituted with 1-2 groups selected from the group consisting of halogen, C₁₋₆ alkyl, -O(C₁₋₆ alkyl), -NH₂, -NH(C₁₋₆ alkyl), -N(C₁₋₆ alkyl)₂;
preferably, R₅ and R₆ are each independently selected from the group consisting of hydrogen, C₁₋₄ alkyl, wherein the alkyl is optionally substituted with 1-2 groups selected from the group consisting of halogen, -O(C₁₋₂ alkyl), -NH(C₁₋₂ alkyl), -N(C₁₋₂ alkyl)₂, five- to seven-membered heterocyclyl, wherein the heterocyclyl contains 1-2 heteroatoms selected from N, O, S, and at least one of the heteroatoms is N, and the heterocyclyl is optionally substituted with 1-2 groups selected from the group consisting of C₁₋₄ alkyl; or
R₅ and R₆ together with the N atom to which they are attached form a five- to seven-membered heterocyclyl, and the heterocyclyl optionally contains 1 additional heteroatom selected from the group consisting of N, O, S, and is optionally substituted with 1-2 groups selected from the group consisting of halogen, C₁₋₄ alkyl, -O(C₁₋₂ alkyl), -NH(C₁₋₂ alkyl), -N(C₁₋₂ alkyl)₂;
more preferably, R₅ and R₆ are each independently selected from the group consisting of hydrogen, C₁₋₂ alkyl, wherein the alkyl is optionally substituted with 1-2 groups selected from the group consisting of methoxy, dimethylamino, morpholinyl, piperidinyl or piperazinyl, wherein the morpholinyl, piperidinyl or piperazinyl is optionally substituted with methyl; or
R₅ and R₆ together with the N atom to which they are attached form a morpholinyl, piperidinyl or piperazinyl, wherein the morpholinyl, piperidinyl or piperazinyl is optionally substituted with methyl;
more preferably, R₅ and R₆ are each independently selected from the group consisting of hydrogen, C₁₋₂ alkyl, wherein the alkyl is substituted with morpholinyl; or
R₅ and R₆ together with the N atom to which they are attached form a morpholinyl group.

6. The method of claim 1, wherein the compound of formula (VII) has the structure of the following compound 5A2, and the method for preparing the compound of formula (VII) comprises:
Step (A): reacting compound 1 with compound B1 to give compound 5A2

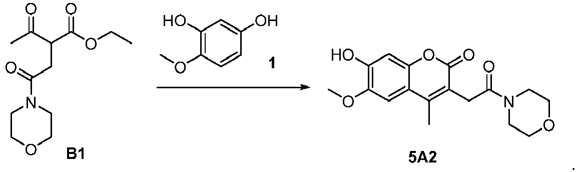

7. The method of any one of claims 1-6, wherein step (A) is carried out under an acidic condition, and/or the reaction temperature is -20 °C to 70 °C, preferably -5 °C to 60 °C, more preferably 20 °C to 50 °C.

8. A method for the preparation of a compound of formula (I), comprising
Step (A): reacting a compound of formula (VI) with a compound of formula (V) to give a compound of formula (VII) 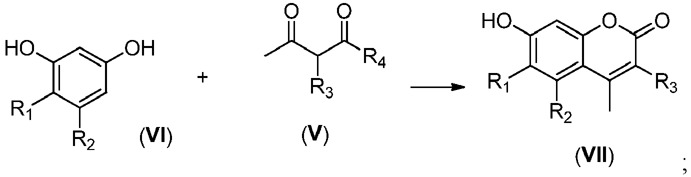 and
Step (B): preparing the compound of formula (I) from the compound of formula (VII) 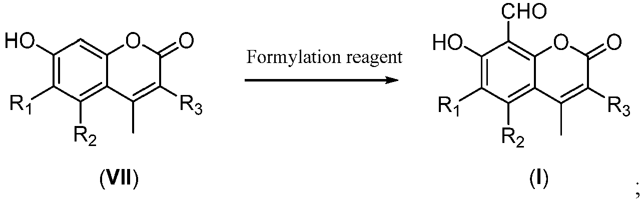
wherein R₁, R₂, R₃ and R₄ are as defined in claim 1.

9. An intermediate compound, wherein the structure is shown in the following formula (V): 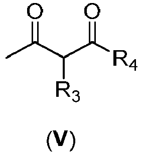 wherein R₃ and R₄ are as defined in any one of claims 1-5.

10. The intermediate compound of claim 9, which is compound B1:

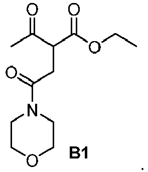

11. The method of claim 1, further comprising, before step (A):
Step (a): condensing the compound of formula (II) with amine compound HNR₅R₆ to give a compound of formula (III) 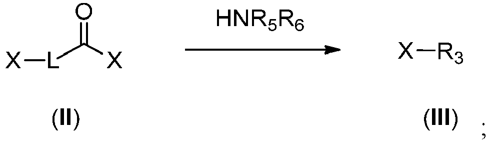
wherein X is halogen, preferably Cl or Br, more preferably Br; L, R₃, R₅ and R₆ are as defined in claim 1;
preferably, the reaction is carried out in an organic solvent; more preferably, the organic solvent is a neutral aprotic solvent; further preferably, the organic solvent is selected from the group consisting of a hydrocarbon solvent, an ether solvent, a disubstituted amide solvent, an ester solvent and a combination thereof;
more preferably,
the hydrocarbon solvent is selected from the group consisting of dichloromethane, toluene, xylene, and a combination thereof; and/or
the ether solvent is selected from the group consisting of tetrahydrofuran, methyltetrahydrofuran, dioxane, ethylene glycol dimethyl ether, methyl tert-butyl ether, and a combination thereof; and/or
the disubstituted amide solvent is selected from *N*,*N*-dimethylformamide, *N,N-*diethylformamide, *N*,*N*-dimethylacetamide, *N*,*N*-diethylacetamide, N-methylpyrrolidone, and a combination thereof; and/or
the ester solvent is selected from the group consisting of ethyl acetate, isopropyl acetate, butyl acetate, and a combination thereof;
and/or
the reaction temperature is -10 °C to 60 °C, preferably 0 °C to room temperature, more preferably 0 °C to 10 °C;
Step (b): reacting the compound of formula (III) with the compound of formula (IV) to give a compound of formula (V) 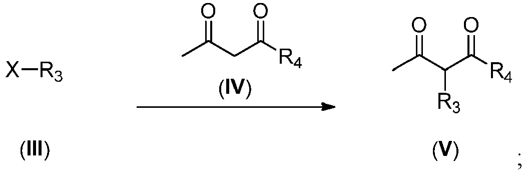 wherein X is halogen, preferably Cl or Br, more preferably Br; R₄ is as defined in claim 1;
preferably, the reaction is carried out in the presence of a base, wherein the base is an organic base or an inorganic base;
more preferably,
the organic base is selected from the group consisting of NaNH₂, sodium alcoholate, K-HMDS, Li-HMDS; and/or
the inorganic base is selected from the group consisting of carbonate and metal hydride; preferably, the carbonate is selected from sodium carbonate, potassium carbonate, lithium carbonate; more preferably sodium carbonate or potassium carbonate, particularly preferably potassium carbonate; and/or the metal hydride is selected from the group consisting of NaH, KH, LiH, CaH₂, particularly preferably NaH;
preferably, the reaction is carried out in an organic solvent; preferably, the organic solvent is a neutral aprotic solvent; further preferably, the organic solvent is selected from the group consisting of a hydrocarbon solvent, an ether solvent, a disubstituted amide solvent, and a combination thereof;
more preferably,
the hydrocarbon solvent is selected from the group consisting of dichloromethane, toluene, xylene, and a combination thereof; and/or
the ether solvent is selected from the group consisting of tetrahydrofuran, methyltetrahydrofuran, dioxane, ethylene glycol dimethyl ether, methyl tert-butyl ether, and a combination thereof; and/or
the disubstituted amide solvent is selected from *N*,*N*-dimethylformamide, *N,N-*diethylformamide, *N*,*N*-dimethylacetamide, *N*,*N*-diethylacetamide, N-methylpyrrolidone, and a combination thereof; and/or
and/or
the reaction temperature is -20 to 100 °C, preferably -20 to 80 °C, more preferably -10 to 60 °C.

12. The method of claim 6, further comprising, before step (A):
Step (a): condensing compound D1 with morpholine to give compound C1 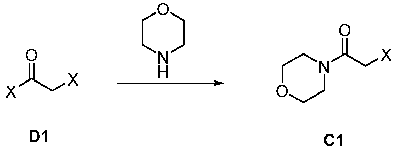
Step (b): reacting compound C1 with ethyl acetoacetate to give compound B 1 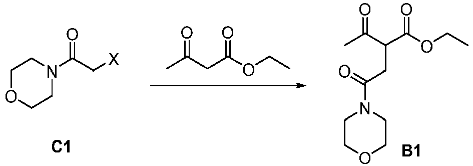 wherein X is halogen, preferably Cl or Br, more preferably Br.

13. A crystal form I of the compound of the following formula, wherein the X-ray powder diffraction pattern of the crystal form I has characteristic peaks at diffraction angles (2θ) of about 8.44±0.2°, 13.11±0.2°, 15.70±0.2°, 19.73±0.2°, 21.00±0.2° and 22.91±0.2°,

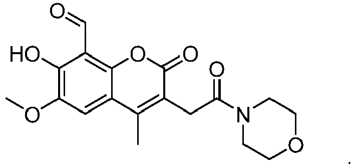

14. The crystal form I of claim 13, wherein the X-ray powder diffraction pattern of the crystal form I has characteristic peaks at diffraction angles (2θ) of about 8.44±0.2°, 10.91±0.2°, 10.68±0.2°, 13.11±0.2°, 15.70±0.2°, 17.54±0.2°, 19.73±0.2°, 21.00±0.2°, 22.91±0.2° and 26.27±0.2°,
preferably, the X-ray powder diffraction pattern of the crystal form I has characteristic peaks at diffraction angles (2θ) of about 8.44±0.2°, 10.91±0.2°, 10.68±0.2°, 13.11±0.2°, 15.70±0.2°, 16.90±0.2°, 17.54±0.2°, 19.73±0.2°, 21.00±0.2°, 22.91±0.2°, 26.27±0.2°, 28.64±0.2°.

15. The crystal form I of claim 13 or 14, wherein the X-ray powder diffraction pattern of the crystal form I has characteristic peaks at diffraction angles (2θ) as shown in Table 1.

16. The crystalline form I of any one of claims 13-15, wherein the X-ray powder diffraction pattern of the crystal form I is substantially as shown in Figure 1.

17. The crystal form I of any one of claims 13-16, wherein the crystal form I satisfies at least one of the following (1) to (3):
(1) The thermogravimetric analysis pattern is substantially as shown in Figure 2;
(2) The differential scanning calorimetry analysis pattern is substantially as shown in Figure 3;
(3) The dynamic vapor sorption pattern is substantially as shown in Figure 4.

18. The crystalline form I of any one of claims 13-17, wherein the crystal form I has an onset temperature of about 239°C ± 5°C and a peak temperature of about 242°C ± 5°C when **characterized by** DSC.

19. The crystal form I of any one of claims 13-18, which is an anhydrate crystal form.

20. A method of preparing the crystal form I of any one of claims 13-19, comprising:
Step i: after the reaction in step (B) is completed, concentrating the reaction solution and adding water and the compound seed crystal of formula (I) to induce crystallization;
Step ii: separating the crude product obtained in step i, stirring with a suitable solvent under a heating condition, then cooling, and isolating the compound of formula (I) in a crystalline state.

21. The method of claim 20, wherein
after separating the crude product and before stirring with a suitable solvent under a heating condition, step ii further comprises the step of rinsing the crude product with a suitable solvent; and/or
after isolating the compound of formula (I) in crystalline state, step ii further comprises the steps of rinsing with a suitable solvent and drying; and/or
the suitable solvent described in step ii is selected from the group consisting of a nitrile solvent or a mixed solvent of a nitrile solvent and water; preferably, the nitrile solvent is selected from the group consisting of acetonitrile, benzonitrile, phenylacetonitrile or a combination thereof, preferably acetonitrile; and/or
the heating temperature in step ii is preferably 40-80 °C, more preferably 50-70 °C; and/or the heating time of step ii is at least 2h, preferably at least 4h, more preferably at least 8h.

22. A pharmaceutical composition comprising the crystal form I of any one of claims 13-19 and one or more pharmaceutically acceptable carriers.

23. Use of the crystal form I of any one of claims 13-19 or the pharmaceutical composition of claim 22 in the manufacture of a medicament for the treatment or prevention of a disease, disorder or condition associated with the unfolded protein response or a disease, disorder or condition associated with a target of regulated IRE1-dependent decay.

24. The use of claim 23, wherein the disease, disorder or condition associated with an unfolded protein response is selected from the group consisting of B cell autoimmune disease, cancer and viral infection; preferably,
the B cell autoimmune disease is selected from the group consisting of Addison's disease, antiphospholipid syndrome, aplastic anemia, autoimmune hemolytic anemias, autoimmune hepatitis, autoimmune hypophysitis, autoimmune lymphoproliferative disorders, autoimmune myocarditis, Churg-Strauss syndrome, epidermolysis bullosa acquisita, giant cell arteritis, Goodpasture's syndrome, Graves disease, Guillain-Barre syndrome, Hashimoto's thyroiditis, idiopathic thrombocytopenic purpura, IgA nephropathy, myasthenia gravis, pemphigus foliaceous, pemphigus vulgaris, polyarteritis nodosa, polymyositis/dermatomyositis, rheumatoid arthritis, scleroderma, Sjögren's syndrome, systemic lupus erythematosus, Takayasu's arteritis and Wegener's granulomatosis; and /or
the cancer is a solid tumor selected from the group consisting of a breast tumor, a bone tumor, a prostate tumor, a lung tumor, an adrenal gland tumor, a bile duct tumor, a bladder tumor, a bronchial tumor, a nervous tissue tumor, a gallbladder tumor, a gastric tumor, a salivary gland tumor, a esophageal tumor, a small intestine tumor, a cervical tumor, a colon tumor, a rectal tumor, a liver tumor, an ovarian tumor, a pancreatic tumor, a pituitary adenoma and secretory adenoma; and/or
the cancer is a drug-resistant or radiation-resistant solid tumor; and/or
the cancer is a hematological tumor selected from the group consisting of a lymphoma, a leukemia and a monoclonal gammopathy of undetermined significance (MGUS); preferably, the lymphoma is selected from multiple myeloma, Hodgkin's lymphoma, non-Hodgkin's lymphoma, and/or the leukemia is selected from acute and chronic types of lymphocytic leukemia and acute and chronic types of myeloid leukemias; and/or
the viral infection is an enveloped virus infection; preferably, the viral infection is an enveloped virus infection that utilizes an unfolded protein response pathway in replicating and forming infectious progeny; more preferably, the viral infection is selected from the group consisting of infections caused by measles virus, pox virus, Ebola virus (Ebola), Epstein Barr virus (EBV), cytomegalovirus (CMV), a virus of the genus Flavivirus, and hepatitis C virus (HCV); wherein the virus of the genus Flavivirus is preferably Japanese encephalitis virus or West Nile virus.

25. The use of claim 23, wherein
the disease, disorder or condition associated with a target of regulated IRE1-dependent decay is selected from the group consisting of a neurological disorder, a disorder involving overproduction of insulin, and a disorder involving inflammation;
preferably, the neurological disorder is schizophrenia, the disorder involving overproduction of insulin is type II diabetes, the disorder involving inflammation is selected from the group consisting of glomerulonephritis, various forms of arthritis, multiple sclerosis and inflammatory bowel disease.
